# EUROPEAN PATENT APPLICATION

(11) **EP 4 364 660 A1**
(43) Date of publication of application: **08.05.2024**
(21) Application number: 22952143.0
(22) Date of filing: 17.09.2022
(51) Int. Cl.: A61B 5/1455

(54) **IN-BLOOD SUBSTANCE CONCENTRATION MEASUREMENT DEVICE, IN-BLOOD SUBSTANCE CONCENTRATION MEASUREMENT METHOD, AND PROGRAM**

(71) Applicant: Light Touch Technology Incorporated, Osaka-shi, Osaka, 540-0029 (JP)
(72) Inventor: YAMAKAWA, Koichi, Osaka-shi, Osaka 540-0029 (JP); OGAWA, Kanade, Osaka-shi, Osaka 540-0029 (JP); YAMAKAWA, Yoko, Osaka-shi, Osaka 540-0029 (JP)
(74) Representative: Grünecker Patent- und Rechtsanwälte PartG mbB
(86) International application number: PCT/JP2022/034859
(87) International publication number: WO 2024/057553

(57) **Abstract**

Provided are: a light emission unit 20 that emits a laser beam to a region of a subject portion Mp0 that includes a measurement-target portion Mp; a photodetector 30 that receives reflected light L2 based on the laser beam L1 and detects an intensity of the reflected light; a first lens 40 that is disposed between the subject portion and the photodetector 30 in a position where the reflected light L2 from a specific region Mp in the subject portion can form a focused image on the photodetector 30; and a measurement controller 60 that, based on the intensity of the reflected light L2, measures a concentration of the substance in blood in the specific region Mp as a concentration of the substance in blood in the measurement-target portion Mp. The light emission unit 20 is capable of selectively emitting: a first laser beam for target measurement that is absorbed by a first substance in blood that is a measurement-target substance; and a second laser beam for reference measurement that is absorbed by a second substance in blood that is a reference substance.

## Description

### [Technical Field]

The present disclosure relates to a device and a method for measuring, by means of a noninvasive measurement method, the concentration of substances that are included in blood flowing through blood vessels of a living body.

### [Background Art]

To prevent and treat lifestyle-related diseases, it is significant that the states of substances in blood, such as the blood sugar level and blood lipid level, be checked on a daily basis. In particular, for patients with diabetes, which is one of such lifestyle-related diseases, it is required that the concentration of glucose in blood be measured and the blood sugar level be managed on a daily basis in order to prevent complications, and an invasive method in which blood is sampled from a patient to chemically analyze the blood has been conventionally performed.

In contrast, in recent years, a simple noninvasive method has been proposed in which the state of blood in a living body is optically measured without sampling blood. For example, Patent Literature 1 discloses a substance-in-blood concentration measurement device that measures the blood glucose concentration using a noninvasive and simple structure, by emitting high-intensity mid-infrared light to a living body via a waveguide and guiding reflected light of the high-intensity mid-infrared light through the waveguide to a photodetector.

### [Citation List]

### [Patent Literature]

[Patent Literature 1] WO 2016/117520

### [Summary of Invention]

### [Technical Problem]

However, there was a problem with the conventional substance-in-blood concentration measurement device disclosed in document 1, in which a waveguide is used, that measurement cannot be performed properly and stably due to a fluctuation in measurement values caused by the depth position of the measurement-target portion that is a measurement target in a living body differing between individuals.

The present disclosure has been made in view of the above problem, and an object thereof is to provide a substance-in-blood concentration measurement device, a substance-in-blood concentration measurement method, and a program that allow accurate measurement to be performed stably regardless of differences between individuals in the depth of the measurement-target portion in a living body.

### [Solution to Problem]

In order to achieve the above-described object, a substance-in-blood concentration measurement device according to one aspect of the present disclosure is a substance-in-blood concentration measurement device that measures a concentration of a substance in blood included in the blood in a subject portion of a living body, the substance-in-blood concentration measurement device including: a light emission unit that emits a laser beam to a region of the subject portion that includes a measurement-target portion; a photodetector that receives reflected light based on the emitted laser beam and detects an intensity of the reflected light; a first lens that is disposed between the subject portion and the photodetector in a position where the reflected light from a specific region in the subject portion can form a focused image on the photodetector; and a measurement controller that, based on the intensity of the reflected light, measures a concentration of the substance in blood in the specific region as a concentration of the substance in blood in the measurement-target portion, the substance-in-blood concentration measurement device being characterized in that the light emission unit is capable of selectively emitting: a first laser beam for a target measurement that is absorbed by a first substance in blood that is a measurement-target substance; and a second laser beam for a reference measurement that is absorbed by a second substance in blood that is a reference substance.

### [Advantageous Effects of Invention]

According to the substance-in-blood concentration measurement device, the substance-in-blood concentration measurement method, and the program pertaining to aspects of the present disclosure, accurate measurement can be performed stably regardless of differences in the measurement target between individuals.

### [Brief Description of Drawings]

FIG. 1 is a schematic diagram illustrating a structure of a substance-in-blood concentration measurement device 1 according to Embodiment 1.
FIGS. 2A and 2B are enlarged cross-section views of portion A of FIG. 1.
FIG. 3 is a schematic diagram illustrating a structure of a light emission unit 20 in the substance-in-blood concentration measurement device 1.
FIG. 4 is a diagram for describing an overview of a light-receiving-side optical path in the substance-in-blood concentration measurement device 1.
FIG. 5 is a schematic diagram illustrating an overview of an optical path from the light emission unit 20 to a photodetector 30 in the substance-in-blood concentration measurement device 1.
FIG. 6 is a schematic diagram for describing an operation for adjusting the length of an optical path in a structure of a substance-in-blood concentration measurement device according to a comparative example conceived of by the inventor.
FIG. 7 is a schematic diagram for describing an operation for adjusting the length of an optical path from a measurement-target portion Mp to the photodetector 30 by the substance-in-blood concentration measurement device 1.
FIG. 8 is a diagram illustrating a relationship between photodetector positions and hemoglobin-concentration measurement values measured using an example of the substance-in-blood concentration measurement device 1.
FIG. 9 is a diagram illustrating a relationship between photodetector positions and glucose-concentration measurement values measured using the example of the substance-in-blood concentration measurement device 1.
FIG. 10A is a schematic diagram for describing an overview of an optical path from the light emission unit 20 to a photodetector PD simulating a subject portion Mp0 in the substance-in-blood concentration measurement device 1, and FIG. 10B is a schematic diagram for describing the same according to a comparative example.
FIG. 11A is a diagram illustrating a fluctuation in substance-in-blood-concentration measurement results in the substance-in-blood concentration measurement device 1, and FIG. 11B is a diagram illustrating the same in a substance-in-blood concentration measurement device according to the comparative example.
FIG. 12 is a diagram illustrating laser-beam emission positions on the surface of a living body in substance-in-blood-concentration measurement by the substance-in-blood concentration measurement device 1.
FIGS. 13A and 13B are diagrams illustrating a fluctuation in substance-in-blood-concentration measurement results in an example of the substance-in-blood concentration measurement device 1 and a comparative example, respectively.
FIG. 14 is a flowchart illustrating one aspect of a substance-in-blood measurement operation by the substance-in-blood concentration measurement device 1.
FIG. 15 is a flowchart illustrating another aspect of the substance-in-blood measurement operation by the substance-in-blood concentration measurement device 1.
FIG. 16 is a flowchart illustrating yet another aspect of the substance-in-blood measurement operation by the substance-in-blood concentration measurement device 1.
FIG. 17 is a schematic diagram illustrating a structure of a conventional substance-in-blood concentration measurement device 1X.

### [Description of Embodiments]

### <<Process Leading to Embodiments of Invention>>

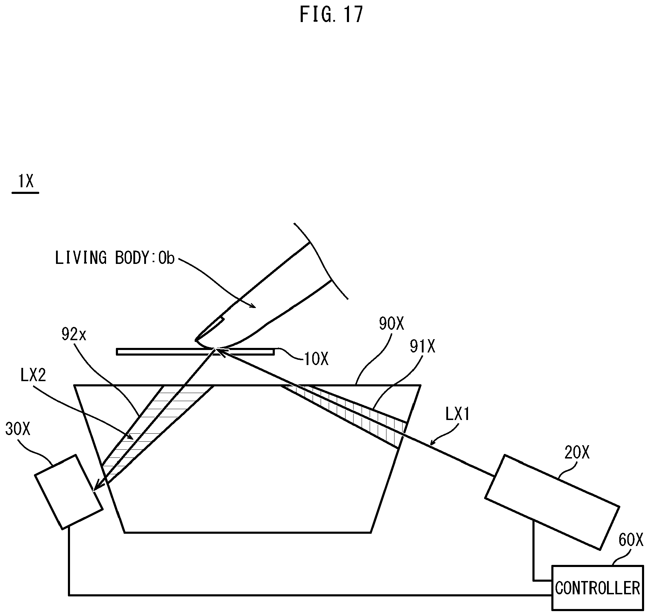

In recent years, a noninvasive substance-in-blood concentration measurement method that does not involve blood sampling has been proposed in order to, inter alia, manage the blood sugar level of diabetes patients on a daily basis. FIG. 17 is a schematic diagram illustrating a structure of a conventional substance-in-blood concentration measurement device 1X (also referred to hereinafter as "device 1X") disclosed in Patent Literature 1, in which the noninvasive method is employed.

As illustrated in FIG. 17, the device 1X includes: a target placement unit 10X on which an examination-target living body Ob is placed; a light emission unit 20X that emits a pulsed laser beam L1 of mid-infrared light; a light guide unit 90X in which an incident-side waveguide 91X and an emission-side waveguide 92X formed from through holes are opened; a photodetector 30X that receives reflected light LX2 reflected from the living body Ob and detects the intensity of the reflected light LX2; and a measurement controller 60X for controlling such elements. Patent Literature 1 indicates that the device 1X can measure the blood glucose concentration using a noninvasive and simple structure by emitting high-intensity mid-infrared light.

However, as mentioned above, experiments by the inventor, etc., revealed that it is difficult to perform measurement properly and stably using the device 1X, in which the noninvasive method is used, because the state of the skin surface of the measurement-target living body, a slight change in the condition of an emitted laser beam, etc., result in a fluctuation in measurement values.

Furthermore, there is a difference among subjects in the depth and position of a portion of the living body inwards of the skin surface that contains blood sugar, which is the substance to be primarily measured, and this can be considered as being a cause of the fluctuation in measurement values.

In order to solve this problem, it would be necessary, for example, to configure the optical system for each individual subject and instance of measurement so as to be suitable for the condition of the subject's body and measurement conditions.

However, a high degree of skill would be required to adjust the optical system appropriately for each individual living body and instance of measurement, and the convenience of the noninvasive method would be impaired to a great extent if such adjustment were to be performed in the measurement of the blood sugar level performed by patients themselves on a daily basis.

In view of this, the inventors, etc., carried out intensive investigation for an optical system structure that would allow accurate measurement to be performed stably in the noninvasive substance-in-blood concentration measurement method regardless of differences between individuals in the depth of the measurement-target portion in a living body and fluctuations in laser-beam emission conditions, and thereby arrived at the following embodiment.

### <<Overview of Embodiments of Invention>>

A substance-in-blood concentration measurement device according to at least one embodiment of the present disclosure is a substance-in-blood concentration measurement device that measures a concentration of a substance in blood included in the blood in a subject portion of a living body, the substance-in-blood concentration measurement device including: a light emission unit that emits a laser beam to a region of the subject portion that includes a measurement-target portion; a photodetector that receives reflected light based on the emitted laser beam and detects an intensity of the reflected light; a first lens that is disposed between the subject portion and the photodetector in a position where the reflected light from a specific region in the subject portion can form a focused image on the photodetector; and a measurement controller that, based on the intensity of the reflected light, measures a concentration of the substance in blood in the specific region as a concentration of the substance in blood in the measurement-target portion, the substance-in-blood concentration measurement device being characterized in that the light emission unit is capable of selectively emitting: a first laser beam for a target measurement that is absorbed by a first substance in blood that is a measurement-target substance; and a second laser beam for a reference measurement that is absorbed by a second substance in blood that is a reference substance.

According to this structure, accurate measurement can be performed stably regardless of differences in the measurement target between individuals.

Furthermore, according to another aspect, in any of the above-described aspects, an absorption rate at which the second laser beam is absorbed by the second substance in blood in the reference measurement is higher than an absorption rate at which the first laser beam is absorbed by the first substance in blood in the target measurement.

Furthermore, according to another aspect, in any of the above-described aspects, a concentration of the reference substance in blood is more stable than a concentration of the measurement-target substance in blood.

According to this structure, the accuracy of the measurement of the measurement target can be improved by performing the target measurement after performing the reference measurement for the reference substance.

Furthermore, according to another aspect, in any of the above-described aspects, the measurement controller is capable of measuring, based on emission of the second laser beam, a concentration of the second substance in blood in a state in which the specific region is included in a blood-vessel region in the subject portion, and is capable of measuring, based on emission of the first laser beam, a concentration of the first substance in blood in the specific region as a concentration of the first substance in blood in the measurement-target portion.

According to this structure, the concentration of the first substance in blood can be measured in a state in which the specific region is included in a blood-vessel region in the subject portion.

Furthermore, according to another aspect, in any of the above-described aspects, a depth of the specific region in the living body can be changed by moving the photodetector along an optical path from the subject portion to the photodetector, and a position of the photodetector can be adjusted along the optical path based on a concentration of the second substance in blood so that the specific region is included in a blood-vessel region in the subject portion.

According to this structure, accurate measurement of the concentration of the first substance in blood can be performed stably at all times regardless of differences, between individuals, in the depth-direction position of blood vessels from the skin surface of the subject, by emitting the first laser beam from the light emission unit at a detector position where reflected light from blood vessels was detected and receiving reflected light from the specific region using the detector.

Furthermore, according to another aspect, in any of the above-described aspects, the light emission unit modulates a wavelength of the laser beam to change a type of substance in blood that can be detected. Alternatively, the light emission unit includes an optical transmitter that emits the first laser beam and an optical transmitter that emits the second laser beam.

According to this structure, multiple types of substances in blood can be measured.

Furthermore, according to another aspect, in any of the above-described aspects, the substance in blood is glucose, and a wavelength of the laser beam is a predetermined wavelength selected from a range of 2.5-12 µm, inclusive. Here, a wavelength of the first laser beam is selected from a range of 6.0-12 µm, inclusive.

According to this structure, the concentration of glucose as the first substance in blood can be measured.

Furthermore, according to another aspect, in any of the above-described aspects, the substance in blood is hemoglobin, and a wavelength of the laser beam is a predetermined wavelength selected from a range of 5.0-12 µm, inclusive.

According to this structure, a blood-vessel region in the subject portion of the living body can be detected, and it can be detected whether or not the specific region that is to become the measurement-target portion in regard to the first substance in blood is included in a blood-vessel region in the subject portion.

Furthermore, according to another aspect, in any of the above-described aspects, a second lens that is positioned between the light emission unit and the subject portion in an optical path of the laser beam, and that condenses the laser beam to the region of the subject portion, and a diaphragm that is positioned between the light emission unit and the second lens are included.

According to this structure, a fluctuation in substance-in-blood-concentration measurement results between instances of measurement can be reduced.

Furthermore, according to another aspect, in any of the above-described aspects, a target placement unit with which a surface of the living body is placed into contact is included, wherein a through-hole is opened in the target placement unit within a region of the target placement unit with which the surface of the living body is placed into contact, the laser beam is emitted to the surface of the living body through the through-hole, and the reflected light is received by the photodetector through the through-hole.

According to this structure, the intensity of the laser beam can be improved because total reflection at the surface of the living body can be suppressed, and also because the laser beam emitted from the light emission unit can directly impinge on the surface of the living body.

Furthermore, according to another aspect, in any of the above-described aspects, a target placement unit with which a surface of the living body is placed into contact is included, wherein a recess portion is formed in the target placement unit within a region of the target placement unit with which the surface of the living body is placed into contact, the laser beam passes through the target placement unit and is emitted to the surface of the living body, and the reflected light passes through the target placement unit and is received by the photodetector.

According to this structure, total reflection at the surface of the living body can be suppressed; furthermore, as a result of there being no opening in the target placement unit, dust, dirt, water vapor, etc., can be prevented from entering the atmosphere in which an optical system including the light emission unit, etc., is present, and the target placement unit can thus be provided with a dust-proofing function.

Furthermore, according to another aspect, in any of the above-described aspects, the measurement-target portion is a blood-vessel region in the subject portion that is located inward of the epidermis, and the first lens transfers a region of the measurement-target portion that is irradiated with the laser beam onto a light receiving surface of the photodetector.

According to this structure, compared to a conventional device in which a waveguide is used, a false-signal (noise) component produced by the reflected light being scattered at the skin surface can be reduced, and the S/N ratio in optical measurement can be improved.

According to this structure, the blood glucose concentration can be stably measured because a laser beam having such a wavelength is absorbed by glucose to a greater extent than near-infrared light and has lower transmittance than near-infrared light, allowing only the skin region to be observed.

Furthermore, according to another aspect, in any of the above-described aspects, the substance in blood is lactic acid, and a wavelength of the laser beam is a predetermined wavelength selected from a range of 5.0-12 µm, inclusive. Here, a wavelength of the first laser beam is within a range of -0.05 µm to +0.05 µm, inclusive, from 5.77 µm, 6.87 µm, 7.27 µm, 8.23 µm. 8.87 µm, or 9.55 µm.

According to this structure, the concentration of lactic acid in blood can be measured. Furthermore, according to another aspect, in any of the above-described aspects, within a section from the target placement unit to the photodetector in an optical path from the subject portion to the photodetector, the reflected light propagates through a space except within a section in which the reflected light passes through the first lens, and, within a section from the light emission unit to the target placement unit in an optical path from the light emission unit to the subject portion, the laser beam propagates through a space except within a section in which the laser beam passes through the second lens.

According to this structure, compared to a conventional device in which a waveguide is used, a false-signal (noise) component produced by the reflected light being scattered at the skin surface can be reduced, and the S/N ratio in optical measurement can be improved.

Furthermore, a substance-in-blood concentration measurement method according to at least one embodiment is a substance-in-blood concentration measurement method for measuring a concentration of a substance in blood included in the blood in a subject portion of a living body, the substance-in-blood concentration measurement method including: emitting, from a light emission unit to an irradiation region in the subject portion that includes a measurement-target portion, a first laser beam for a target measurement that is absorbed by a first substance in blood that is a measurement-target substance; forming a focused image on a photodetector from reflected light of the first laser beam reflected from a specific region in the subject portion using a first lens positioned between the subject portion and the photodetector; and performing the target measurement by receiving the reflected light of the first laser beam using the photodetector, and measuring a concentration of the first substance in blood based on the reflected light as a concentration of the first substance in blood in the measurement-target portion, wherein, prior to the target measurement, a reference measurement is performed by: emitting, from the light emission unit to the irradiation region, a second laser beam for the reference measurement that is absorbed by a second substance in blood that is a reference substance; forming a focused image on the photoreceptor from reflected light of the second laser beam reflected from the specific region using the first lens; and receiving the reflected light of the second laser beam using the photodetector, and measuring a concentration of the second substance in blood based on the reflected light as a concentration of the second substance in blood in the measurement-target portion.

According to this structure, a substance-in-blood concentration measurement method can be provided according to which accurate measurement can be performed stably regardless of differences in the measurement target between individuals.

Furthermore, according to another aspect, in any of the above-described aspects, an absorption rate at which the second laser beam is absorbed by the second substance in blood in the reference measurement is higher than an absorption rate at which the first laser beam is absorbed by the first substance in blood in the target measurement.

Furthermore, according to another aspect, in any of the above-described aspects, a concentration of the reference substance in blood is more stable than a concentration of the measurement-target substance in blood.

According to this structure, the accuracy of the measurement of the measurement target can be improved by performing the target measurement after performing the reference measurement for the reference substance having a relatively high absorption rate.

Furthermore, according to another aspect, in any of the above-described aspects, prior to the detection of the concentration of the first substance in blood, by further measuring the concentration of the second substance in blood while changing a position of the photodetector along an optical path from the subject portion to the photodetector, the position of the photodetector is adjusted along the optical path so that the specific region is included in a blood-vessel region in the subject portion.

According to this structure, a method can be provided in which accurate measurement of the concentration of the first substance in blood is performed stably at all times regardless of differences, between individuals, in the depth-direction position of blood vessels from the skin surface of the subject by emitting the first laser beam from the light emission unit at a detector position where reflected light from blood vessels was detected and receiving reflected light from the specific region using the detector.

Furthermore, according to another aspect, in any of the above-described aspects, the first laser beam and the second laser beam are emitted to the irradiation region using: a second lens that is positioned between the light emission unit and the subject portion in an optical path from the light emission unit to the subject portion, and that condenses the first laser beam and the second laser beam to the irradiation region; and a diaphragm that is positioned between the light emission unit and the second lens and that restricts light emitted from the light emission unit.

According to this structure, a fluctuation in substance-in-blood-concentration measurement results between instances of measurement can be reduced.

Furthermore, according to another aspect, in any of the above-described aspects, a surface of the living body is placed into contact with a target placement unit, the first laser beam and the second laser beam are emitted to the surface of the living body through a through-hole opened in the target placement unit, and the reflected light is received by the photodetector through the through-hole.

According to this structure, the intensity of the laser beams can be improved because the laser beams emitted from the light emission unit can directly impinge on the surface of the living body.

Furthermore, a program according to at least one embodiment is a program that causes a computer to execute substance-in-blood concentration measurement processing of measuring a concentration of a substance in blood included in the blood in a subject portion of a living body, wherein the substance-in-blood concentration measurement processing includes: emitting, from a light emission unit to an irradiation region in the subject portion that includes a measurement-target portion, a first laser beam for a target measurement that is absorbed by a first substance in blood that is a measurement-target substance; forming a focused image on a photodetector from reflected light of the first laser beam reflected from a specific region in the subject portion using a first lens positioned between the subject portion and the photodetector; and performing the target measurement by receiving the reflected light of the first laser beam using the photodetector, and measuring a concentration of the first substance in blood based on the reflected light as a concentration of the first substance in blood in the measurement-target portion, wherein, prior to the target measurement, a reference measurement is performed by: emitting, from the light emission unit to the irradiation region, a second laser beam for the reference measurement that is absorbed by a second substance in blood that is a reference substance; forming a focused image on the photodetector from reflected light of the second laser beam reflected from the specific region using the first lens; and receiving the reflected light of the second laser beam using the photodetector, and measuring a concentration of the second substance in blood based on the reflected light as a concentration of the second substance in blood in the measurement-target portion.

According to this structure, a program can be provided that allows accurate measurement to be performed stably regardless of differences in the measurement target between individuals.

### <<Embodiment 1»

A substance-in-blood concentration measurement device 1 according to the present embodiment will be described with reference to the drawings. Here, in this description, the positive and negative directions in the height direction may be respectively referred to as the "upward" and "downward" directions, and a surface facing the positive direction in the height direction and a surface facing the negative direction in the height direction may be respectively referred to as a "front" surface and a "rear" surface. Furthermore, members in the drawings are not necessarily drawn to scale. Furthermore, in this description, numerical ranges indicated using the symbol "-" include the values at both ends of the numerical ranges. Further, only preferred examples of materials, numerical values, etc., are indicated in the present embodiment, and there is no limitation thereto.

### <Overall Structure >

The substance-in-blood concentration measurement device 1 (also referred to hereinafter as a "device 1") is a medical device that noninvasively measures the concentration of a substance in the blood of a living body Ob in a measurement-target portion Mp by emitting a laser beam having a specific wavelength from a light source to the measurement-target portion Mp and detecting the intensity of reflected light from the measurement-target portion Mp. As the laser beam, light of a specific wavelength capable of being absorbed by the substance to be measured is used. When the concentration of the substance in blood is high, the intensity of reflected light reflected from the measurement-target portion Mp decreases due to absorption by the substance; thus, the device 1 measures the concentration of the substance in blood by measuring the intensity of reflected light using a photodetector.

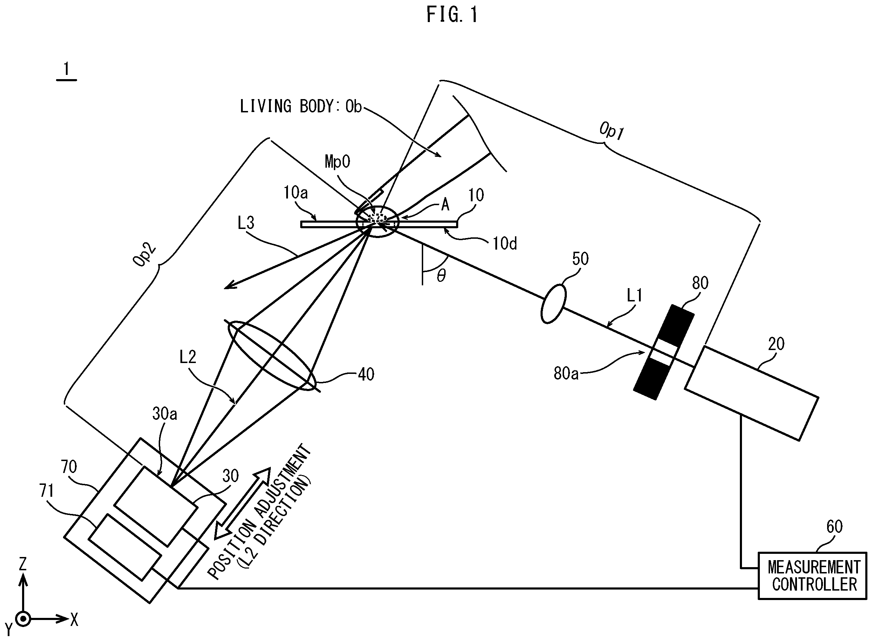

FIG. 1 is a schematic diagram illustrating a structure of the device 1 according to Embodiment 1. As illustrated in FIG. 1, the device 1 includes a target placement unit 10, a light emission unit 20, a photodetector 30, a condenser lens 50, an imaging lens 40, a measurement controller 60, a light detection unit 70, and a diaphragm 80.

Structures of the elements of the device 1 will be described below.

### <Structures of Elements>

### (Target Placement Unit 10)

The target placement unit 10 is a plate-shaped guide member for regulating the measurement-target portion Mp included in a subject portion Mp0 of the living body Ob to a predetermined position and angle that are suitable for measurement when the skin surface of the living body Ob is placed into contact with a front surface 10a of the target placement unit 10. By covering an optical system including the light emission unit 20, etc., with a housing (unillustrated) and providing the target placement unit 10 on the outer-shell portion of the housing, the target placement unit 10 can be made to function as an emission window from which a laser beam L1 is emitted.

The target placement unit 10 is formed from a material such as ZnSe that is transparent with respect to mid-infrared light that is the specific wavelength used for measurement, and may have an antireflection coating layer provided on the front surface thereof. A measurement position is marked on the front surface 10a of the target placement unit 10. By placing the living body Ob into contact with the front surface 10a of the target placement unit 10 with a predetermined pressure in a state in which the living body Ob including the measurement-target portion Mp is positioned with respect to the measurement position, the measurement-target portion Mp, which is a portion of the living body that is located inward of the epidermis, such as the dermis for example, can be kept in a state in which the measurement-target portion Mp is separated from the front surface 10a of the target placement unit 10 by a predetermined distance.

Furthermore, the target placement unit 10 is disposed so that the laser beam L1 emitted from the light emission unit 20 enters from a rear surface 10d-side thereof, and the angle of the front surface 10a relative to the light emission unit 20 is regulated so that an incident angle θ of an incident-side optical axis L1 equals a predetermined angle. Here, the incident angle θ indicates the angle of the optical axis L1 with respect to the normal to the front surface 10a of the target placement unit 10, on which the living body Ob is placed.

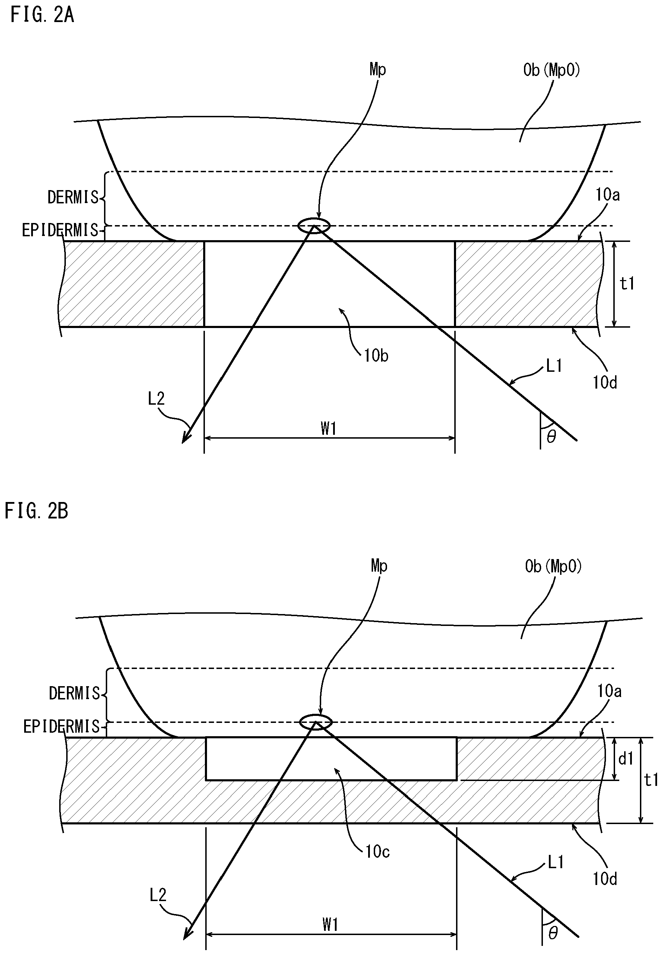

FIGS. 2A and 2B are enlarged cross-sectional views illustrating structures of portion A of FIG. 1. The enlarged diagrams illustrate the portion where the subject portion Mp0 of the living body Ob is placed into contact with the target placement unit 10. As illustrated in FIG. 2A, an opening 10b may be formed in the front surface 10a of the target placement unit 10 within a region of the front surface 10a that comes into contact with the surface of the living body Ob. Due to the opening 10b, the laser beam L1 emitted from the light emission unit 20 is emitted to the surface of the living body Ob through the through-hole 10b opened in the target placement unit 10. Furthermore, as a result of the opening 10b being provided, an air layer can be formed in the portion where the front surface 10a of the target placement unit 10 and the living body Ob come into contact with another, and, compared to a case in which the living body Ob is placed into contact with the target placement unit 10, total reflection at the surface of the living body Ob can be suppressed. Furthermore, by providing the opening 10b, the living body Ob can be placed into closer contact with the front surface 10a of the target placement unit 10 in the circumference of the opening 10b in the front surface 10a. In the present embodiment, as one example, the thickness of the target placement unit 10 may be 500 µm, and the width of the opening 10b may be 700 µm.

Alternatively, as illustrated in FIG. 2B, a recess portion 10c that forms a gap between the front surface 10a of the target placement unit 10 and the living body Ob may be formed in the front surface 10a. In this case, the laser beam L1 emitted from the light emission unit 20 passes through the bottom-surface portion of the recess portion 10c in the target placement unit 10 and is emitted to the surface of the living body Ob. As a result of there being no opening in the target placement unit 10, dust, dirt, water vapor, etc., can be prevented from entering the atmosphere in which an optical system including the light emission unit 20, etc., is present, and the target placement unit 10 can thus be provided with a dust-proofing function.

In this case as well, similarly to the case in which the opening 10b is provided, an air layer is formed between the front surface 10a of the target placement unit 10 and the living body Ob as a result of the recess portion 10c being provided, and, compared to a case in which the living body Ob is placed into contact with the target placement unit 10, total reflection at the surface of the living body Ob can be suppressed. Furthermore, by providing the recess portion 10c, the living body Ob can be placed into closer contact with the front surface 10a of the target placement unit 10 at portions other than the recess portion. According to the present embodiment, as one example, the thickness of the target placement unit 10 may be approximately 500 µm, the width of the recess portion 10c may be approximately 700 µm, and a depth d1 of the recess portion 10c, or in other words the thickness of the air layer, may be approximately 400 µm.

### (Light Emission Unit 20)

The light emission unit 20 is a light source that emits a laser beam having a specific wavelength to the living body, i.e., the subject portion Mp0 of the living body Ob. The light emission unit 20 is capable of emitting a laser beam for target measurement (may be referred to hereinafter as a "first laser beam") that oscillates at a wavelength that is absorbed by a first substance in blood that is the measurement-target substance (may be referred to hereinafter as a "first substance in blood").

Furthermore, in the device 1, the light emission unit 20 is capable of modulating laser-beam wavelength to selectively emit the laser beam for target measurement and a laser beam for reference measurement (may be referred to hereinafter as a "second laser beam") that oscillates at a wavelength that is absorbed by a second substance in blood that is a reference substance (may be referred to hereinafter as a "second substance in blood"). Thus, the type of substance in blood that can be detected is changed.

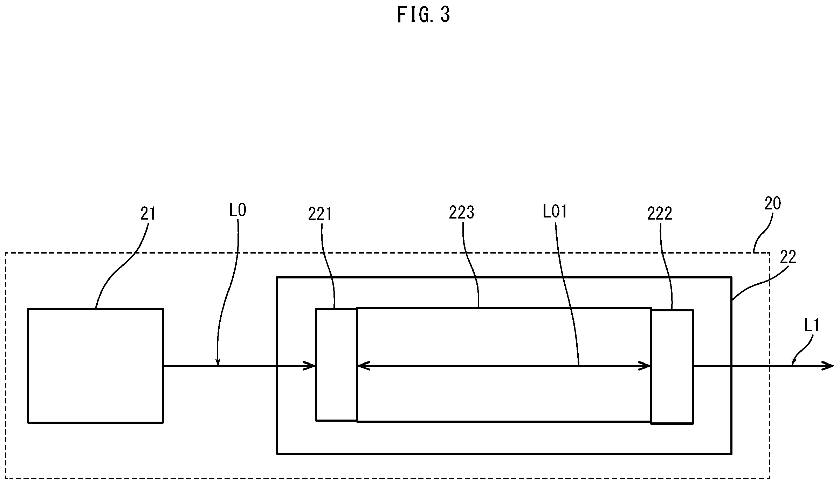

FIG. 3 is a schematic diagram illustrating a structure of the light emission unit 20 in the substance-in-blood concentration measurement device 1. As illustrated in FIG. 2, the light emission unit 20 includes a light source 21 that oscillates pulsed pump light L0 having a shorter wavelength than mid-infrared light, and an optical parametric oscillator (OPO) 22 that converts the wavelength into a longer wavelength and amplifies the pump light L0 to emit the resultant light as the laser beam L1. In the OPO 22, as a result of the pump light L0 being input to an internal nonlinear optical crystal, light of two different wavelengths is oscillated, resulting in signal light having a shorter wavelength and idler light having a longer wavelength being generated. The light emission unit 20 outputs the idler light out of the two types of light as the laser beam L1 to a later stage where the laser beam L1 is used to measure the blood sugar level. A configuration disclosed in a known document, e.g., JP 2010-281891, may be used for the OPO 22.

In the present embodiment, as one example, glucose may be adopted, for example, as the first substance in blood that is the measurement-target substance. In such a case, light having a wavelength selected from mid-infrared light is emitted as the laser beam; i.e., as the wavelength oscillated by optical parametric oscillation, mid-infrared light is used due to being a wavelength that is absorbed to a greater extent by glucose than conventionally used near-infrared light, and a predetermined wavelength selected from the range of 2.5-12 µm, inclusive, may be adopted. A predetermined wavelength selected from the range of 6.0-12 µm, inclusive, is more preferable. Specifically, in the present embodiment, the selected wavelength is 9.26 µm. For example, a wavelength within the range of 9.26 ± 0.05 µm (9.21-9.31 µm, inclusive) may be adopted. Alternatively, the wavelength may be selected from within the range of -0.05 µm to +0.05 µm, inclusive, from 7.05 µm, 7.42 µm, 8.31 µm, 8.7 µm, 9.0 µm, 9.57 µm, 9.77 µm, 10.04 µm, or 10.92 µm.

Thus, the glucose concentration in the subject's blood can be measured as the blood sugar level. In this case, it is necessary to measure the glucose concentration in blood vessels in the skin, and mid-infrared light, which does not readily penetrate deeply into the body, is emitted directly to blood vessels (capillary vessels) in the skin. Mid-infrared light has lower transmittance into the body than near-infrared light conventionally used to measure the blood sugar level; thus, by identifying the position of blood vessels in the skin and emitting mid-infrared light thereto, an effect can be obtained that observation of only the blood-vessel portion is possible, in which case the observation is less susceptible to the influence of other body components present deeper in the body. Furthermore, by using mid-infrared light, an effect can also be obtained that the negative influence of overlaps of combination tones and overtones of fundamental vibrations on the measurement is reduced, and glucose can be measured more accurately than with near-infrared light.

On the other hand, as the reference substance (second substance in blood) measured in the reference measurement, a substance in blood satisfying the following is selected. That is, the substance has a higher laser-beam absorption rate than the measurement-target substance and thus has high measurement sensitivity, and the concentration of the substance in blood is very stable, resulting in less fluctuation in measurement results.

That is, the absorption rate at which the laser beam in the reference measurement is absorbed by the reference substance (second substance in blood) is preferably higher than the absorption rate at which the laser beam for target measurement is absorbed by the measurement-target substance (first substance in blood) in the target measurement. Additionally/alternatively, the concentration of the reference substance (second substance in blood) in blood is preferably more stable than the concentration of the measurement-target substance (first substance in blood) in blood.

The accuracy of the measurement of the measurement target can be improved by performing the target measurement after performing the reference measurement for the reference substance having this structure.

In the present embodiment, hemoglobin can be selected as one example of the reference substance (second substance in blood). By detecting hemoglobin in blood vessels, the positions of capillary vessels in the living body can be detected, and the most-suitable position of the measurement-target portion Mp in the subject portion Mp0 of the living body Ob for performing the measurement of the measurement-target substance (first substance in blood) can be specified.

When hemoglobin is adopted as the first substance in blood or the second substance in blood, the laser beam to be emitted for measurement is light having a wavelength selected from mid-infrared light, and the wavelength is a predetermined wavelength selected from the range of 5.0-12 µm, inclusive. Specifically, for example, a wavelength within the range of 8.00 ± 0.1 µm (7.9-8.1 µm, inclusive) may be adopted. Alternatively, the wavelength may be selected from: the range of 5.26-6.76 µm, inclusive; within the range of -0.1 µm to +0.1 µm, inclusive, from 7.17 µm; within the range of -0.1 µm to +0.1 µm, inclusive, from 7.58 µm; the range of 7.58-8.33 µm, inclusive; or within the range of-0.1 µm to +0.1 µm, inclusive, from 8.55 µm.

The light source 21 may include a Q-switched Nd:YAG laser (oscillation wavelength: 1.064 µm) or a Q-switched Yb:YAG laser (oscillation wavelength: 1.030 µm). Thus, the light source 21 can oscillate pulsed pump light L0 having a shorter wavelength than mid-infrared light. For example, the pump light L0 may have a pulse width of approximately 8 ns and a frequency of 10 Hz or higher. Furthermore, because a Q-switched Nd:YAG laser or Yb:YAG laser operates as a passive Q-switch that switches passively using a saturable absorber, the light source 21 can be simplified and reduced in size.

As illustrated in FIG. 3, the OPO 22 includes an incident-side semi-transmissive mirror 221, an output-side semi-transmissive mirror 222, and a nonlinear optical crystal 223, and the nonlinear optical crystal 223 is disposed inside an optical resonator formed by disposing the incident-side semi-transmissive mirror 221 and the output-side semi-transmissive mirror 222 so as to face one another. Light L01 passing through the incident-side semi-transmissive mirror 221 enters the nonlinear optical crystal 223 and is converted into light having a wavelength determined by the nonlinear optical crystal 223, and is also optically parametrically amplified between the incident-side semi-transmissive mirror 221 and the output-side semi-transmissive mirror 222. The amplified light passes through the output-side semi-transmissive mirror 222 and is output as the laser beam L1.

In the nonlinear optical crystal 223, AgGaS suitable for wavelength conversion is used under phase-matching conditions. The wavelength of the oscillated laser beam L1 can be adjusted by adjusting the type and matching conditions of the nonlinear optical crystal 223. GaSe, ZnGeP₂, CdSiP₂, LiInS₂, LiGaSe₂, LiInSe₂, LiGaTe₂, etc., may be used as the nonlinear optical crystal. The laser beam L1 emitted from the OPO 22 is provided with a repetition frequency corresponding to the pump light L0, e.g., a pulse width of approximately 8 ns, and a high-intensity peak output of 10 W to 1 kW, inclusive, can be realized by the short pulse width.

As a result of the light source 21 and the OPO 22 being used in the light emission unit 20 as described above, the laser beam L1, which has a high intensity that is approximately 10³ to 10⁵ times the intensity of a conventional light source such as a quantum cascade laser, can be obtained.

A device in which the wavelength of light emitted by the light emission unit 20 is changed can be realized by either: changing the configuration of the oscillation wavelength of the OPO 22 in the light emission unit 20 and changing the phase matching conditions of the nonlinear crystal 223 in the OPO 22; or changing the OPO 22 to that in which the phase matching conditions of the nonlinear crystal 223 are different.

According to this structure, the blood sugar level can be measured using mid-infrared light having low transmittance into the body.

The light emission unit 20 is electrically connected to the later-described measurement controller 60, and outputs the laser beam L1 based on a control signal from the measurement controller 60.

### (Condenser Lens 50)

As illustrated in FIG. 1, the condenser lens 50 (also referred to hereinafter as a "second lens") for condensing emitted light to a specific region (measurement-target portion Mp) in the subject portion Mp0 of the living body Ob is disposed on an optical path Op1 of the laser beam L1 from the light emission unit 20 to the subject portion Mp0 of the living body Ob. Within a section of the optical path Op1 from the light emission unit 20 to the surface of the living body Ob (in a case in which the structure in FIG. 2A is adopted) or a section of the optical path Op1 from the light emission unit 20 to the rear surface 10d of the target placement unit 10 (in a case in which the structure in FIG. 2B is adopted), the laser beam L1 propagates through a space filled with a gas, etc., for example, except within a section in which the laser beam L1 passes through the condenser lens 50.

The condenser lens 50 is optically designed so that the laser beam L1 emitted from the light emission unit 20 is condensed to a depth corresponding to a portion of the living body located inward of the epidermis, such as the dermis for example. The depth corresponds to the specific region that is to become the measurement-target portion Mp of the living body Ob, which is separated by a predetermined distance from the front surface 10a of the target placement unit 10. The incident angle θ of the laser beam L1 on the specific region that is to become the measurement-target portion Mp is determined by the angle of the light emission unit 20 relative to the front surface 10a of the target placement unit 10 and the refraction angle of the laser beam L1 having entered the target placement unit 10. In the present embodiment, the incident angle θ may be set to 45 degrees or more, and may further be set within the range of 60-70 degrees, inclusive, for example.

Here, a beam splitter (unillustrated) formed from a semi-transmissive mirror may be disposed between the light emission unit 20 and the condenser lens 50 to split a portion of the laser beam L1 as a reference signal, and the change in intensity of the laser beam L1 may be detected using a photodetector for monitoring (unillustrated) and be used in detection-signal normalization processing by the photodetector 30. This enables the output of the photodetector 30 to be compensated based on the change in intensity of the laser beam L1.

The laser beam L1 having passed through the condenser lens 50 passes through the target placement unit 10 and enters the living body Ob, where the laser beam L1 passes through the epithelial interstitial tissue of the living body to be scattered or diffusely reflected. The reflected light L2 so produced passes through the target placement unit 10 once again and is emitted toward the photodetector 30.

### (Diaphragm 80)

The diaphragm 80 is disposed in a section between the light emission unit 20 and the condenser lens 50 of the optical path Op1 of the laser beam L1 from the light emission unit 20 to the subject portion Mp0 of the living body Ob. The diaphragm 80 is formed from a light-blocking plate-shaped member, and an opening 80a (aperture) is opened in the center portion thereof.

The center of the opening 80a is aligned with the optical axis of the laser beam L1. Furthermore, the beam diameter of the laser beam L1 may be restricted to approximately one third by the opening 80a, for example. Furthermore, the diaphragm 80 may be disposed at a position where the distance between the condenser lens 50 and the light emission unit 20 is internally divided to a:b, where 1/f = 1/a + 1/b holds true, and f is the focal length of the condenser lens 50.

By providing the diaphragm 80 between the light emission unit 20 and the condenser lens 50 in the optical path Op1 in such a manner, the fluctuation in the emission position of the laser beam L1 emitted to the living body Ob can be reduced.

### (Imaging Lens 40)

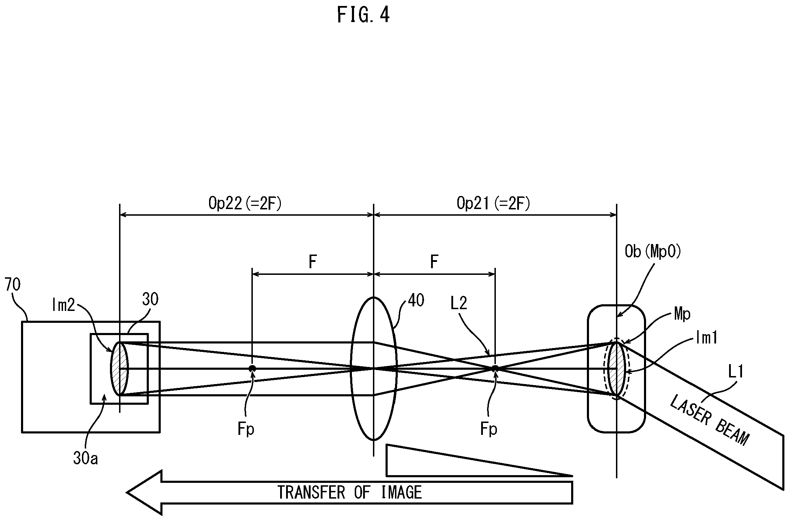

FIG. 4 is a diagram for describing an overview of a light-receiving-side optical path in the substance-in-blood concentration measurement device 1, and is a schematic diagram illustrating the subject portion Mp0 of the living body Ob and a screen 30a of the photodetector 30 as seen in a cross-sectional view. As illustrated in FIGS. 1 and 4, on an optical path Op2 of the reflected light L2 from the subject portion Mp0 of the living body Ob to the photodetector 30, the imaging lens 40 (also referred to hereinafter as a "first lens"), which causes the reflected light L2 having been diffusely reflected in the specific region of the subject portion Mp0 to form a focused image on the photodetector 30, is disposed. Within a section of the optical path Op2 from the surface of the living body Ob including the subject portion Mp0 to the photodetector 30 (in a case in which the structure in FIG. 2A is adopted) or a section of the optical path Op2 from the rear surface 10d of the target placement unit 10 to the photodetector 30 (in a case in which the structure in FIG. 2B is adopted), the reflected light L2 propagates through a space, except within a section in which the reflected light L2 passes through the imaging lens 40.

The imaging lens 40 is optically designed such that an image Im1 of the specific region corresponding to the measurement-target portion Mp in the subject portion Mp0 is diffusely reflected, and the image Im1 becoming the reflected light L2 is imaged as a focused image Im2 on the screen 30a of the photodetector 30 by the imaging lens 40.

In the present embodiment, a distance Op21 between the center of the imaging lens 40 and the specific region (measurement-target portion Mp) of the living body Ob and a distance Op22 between the screen 30a of the photodetector 30 and the center of the imaging lens 40 are equivalent. Thus, a positional relationship is realized such that the image Im1 at a depth corresponding to the specific region (measurement-target portion Mp) in the subject portion Mp0 irradiated with mid-infrared light is transferred onto the screen 30a of the photodetector 30 as the image Im2 of equivalent size.

Here, for example, the specific region (measurement-target portion Mp) is preferably located in a portion of the living body that is located inward of the epidermis (may be referred to hereinafter as a "a portion of the living body inwards of the skin surface" or "an inwards portion of the living body"), such as the dermis for example. Here, the distances Op21 and Op22 may both be 2F, where F is the focal length of focal points Fp of the imaging lens 40.

However, the lengths of the distances Op21 and Op22 are not limited to those described above; the factors applied to the distances Op21 and Op22 may be set so that the image Im1 irradiated with mid-infrared light fits precisely in the screen 30a of the photodetector 30, and an imaging lens 40 achieving such factors may be configured.

The angle of incidence of the reflected light L2 on the imaging lens 40 is determined by the angle of the imaging lens 40 relative to the front surface 10a of the target placement unit 10 and the refraction angle of the reflected light L2 emitted from the target placement unit 10. In the present embodiment, the incident angle may be set to 0-40 degrees, inclusive, and more preferably 20-3 0 degrees, inclusive, for example.

### (Light Detection Unit 70)

In the substance-in-blood concentration measurement device 1, in a state in which the second laser beam for reference measurement is emitted from the light emission unit 20, the detector 30 is moved along the optical path Op2 from the subject portion Mp0 to the photodetector 30 to change the distance of the photodetector 30 from the imaging lens 40. Thus, as illustrated in FIG. 1, the light detection unit 70 includes the photodetector 30 and a movable mechanism 71. Structures of these elements will be described below.

### (Photodetector 30)

The photodetector 30 is a near-infrared/mid-infrared sensor that receives reflected light from the specific region (measurement-target portion Mp) that is based on the emitted laser beam L1, and detects the intensity of the reflected light. The photodetector 30 outputs an electric signal corresponding to the intensity of the received reflected light. For example, as the photodetector 30, a single-element infrared sensor that outputs the intensity of the reflected light as a one-dimensional voltage value may be used.

In the substance-in-blood concentration measurement device 1, the photodetector 30 can receive reflected light having sufficiently high intensity relative to background light as a result of the intensity of the emitted laser beam L1 being increased by the light emission unit 20 and the reflected light reflected from the measurement-target portion Mp being imaged on the photodetector 30 by the imaging lens 40; thus, a high signal-to-noise (S/N) ratio is realized and measurement can be performed with high accuracy. Because the laser beam L1 and the reflected light L2 are monochromatic and have high intensity as discussed above, the photodetector 30 only needs to perform processing of detecting light intensity, and does not need to execute multivariate analysis, spectrum analysis, etc., based on wavelength sweeping as in a photoacoustic optical method in which a quantum cascade laser is used. Thus, the accuracy required in the detection is relaxed, and the easily usable electronic cooling method, etc., can also be used.

Note that, as the photodetector 30, an HgCdTe infrared detector cooled with liquid nitrogen may be used, for example. In this case, the light intensity of the reflected light L2 can be detected with a higher S/N ratio by cooling the photodetector 30 to approximately 77 K using liquid nitrogen.

The photodetector 30 is electrically connected to the later-described measurement controller 60, and, based on a control signal from the measurement controller 60, outputs the intensity of received reflected light to the measurement controller 60 as a one-dimensional voltage value.

### [Movable Mechanism 71]

The movable mechanism 71 is a linear transportation mechanism that can reversibly move the detector 30 along the optical path Op2 from the subject portion Mp0 to the photodetector 30. As the movable mechanism 71, a general-purpose linear transportation mechanism such as a linear motor, a ball screw, or a rack-and-pinion mechanism can be used. The movable mechanism 71 is electrically connected to the later-described measurement controller 60, and, based on a control signal supplied from the measurement controller 60, transports the detector 30 to a predetermined position.

### (Measurement Controller 60)

The measurement controller 60 is electrically connected to the light emission unit 20, the photodetector 30, and the movable mechanism 71: The circuitry drives the light source 21 of the light emission unit 20 to cause the light source 21 to oscillate the pulsed pump light L0, and detects the light intensity of the reflected light L2 based on an output signal from the photodetector 30 to calculate the concentration of a substance in blood in the specific region of the subject portion Mp0.

Also, the output from the photodetector for monitoring may be input to the measurement controller 60, and, even if the intensity of the laser beam L1 emitted from the light emission unit 20 changes, the measurement controller 60 may calculate the concentration of a substance in blood while compensating for the influence of the change in the intensity of the laser beam L1 by normalizing the output from the photodetector 30 using the output from the photodetector for monitoring, as described above.

Furthermore, the measurement controller 60 outputs a control signal to the movable mechanism 71 to drive the movable mechanism 71 and transport the detector 30 along the optical path Op2 to a predetermined position.

The measurement controller 60 executes the later-described substance-in-blood concentration measurement processing based on a predetermined program and executes the above-described processing, such as the transportation of the detector 30, the emission of the laser beam L1 from the light emission unit 20, and the calculation of the concentration of a substance in blood based on a signal from the photodetector 30.

### <Effects Achieved by Substance-In-Blood Concentration Measurement Device 1>

### (Improvement of S/N ratio)

An improvement in S/N ratio during measurement achieved by the optical system in the device 1 will be described.

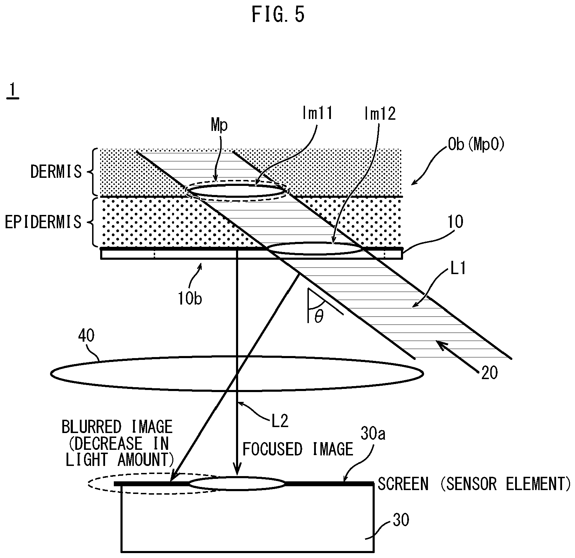

FIG. 5 is a schematic diagram illustrating an overview of an optical path from the light emission unit 20 to the photodetector 30 in the device 1. As illustrated in FIG. 5, in the device 1, the laser beam L1 emitted from the light emission unit 20 enters the living body Ob at the incident angle θ along the optical path toward the specific region (measurement-target portion Mp) in the subject portion Mp0 and is absorbed by substances in blood; this results in a reflected light component (Im12) from the skin surface being produced in addition to a reflected light component (Im11) from the specific region (measurement-target portion Mp) in a portion of the living body inwards of the skin surface.

However, in the device 1, a focused image is formed by the imaging lens 40 on the screen 30a of the photodetector 30 mainly from the reflected light component (Im11) among these reflected light components (Im11, Im12), which is from the specific region (measurement-target portion Mp) in the inwards portion of the living body.

The reflected light component (Im12) from the skin surface enters the imaging lens 40; however, because the angle of incidence of the reflected light component (Im12) on the imaging lens 40 is different from that of the reflected light component (Im11) reflected in the inwards portion of the living body, the reflected light component (Im12) is guided to the outside of the area of the screen 30a of the photodetector 30. Otherwise, even if the reflected light component (Im12) is guided to within the area of the screen 30a of the photodetector 30, the reflected light component (Im12) is not focused (is blurred). Thus, the light amount thereof decreases and the signal intensity detected by the photodetector 30 decreases.

Due to this, the reflected light component (Im12) from the skin surface, which is detected as noise, does not affect the optical measurement much.

That is, in the device 1, the reflected light component (Im11) from the specific region (measurement-target portion Mp) that is to be mainly measured and located in the portion of the living body inwards of the skin surface forms a focused image on the screen 30a of the photodetector 30 and is reflected in the optical measurement by the photodetector 30; thus, a measurement result that is highly correlated with a blood-sugar-level measurement result based on self-monitoring-of-blood-glucose (SMBG) and that has high reproducibility can be obtained.

In such a manner, a false-signal (noise) component produced by reflected light scattered at the skin surface can be reduced and the S/N ratio in optical measurement can be improved with the device 1, compared to the conventional device 1X, in which waveguides are used.

### (Detection of Blood-Vessel Region in Subject Portion Mp0)

An improvement in measurement accuracy based on the detection of a blood-vessel region in the subject portion Mp0 using the optical system in the device 1 will be described.

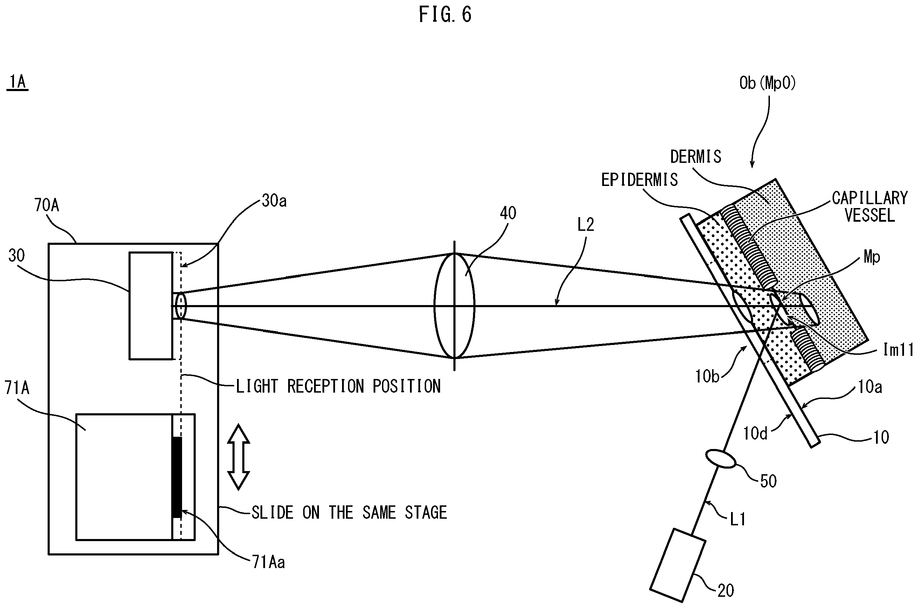

FIG. 6 is a schematic diagram for describing a similar operation for adjusting the length of an optical path in a structure of a substance-in-blood concentration measurement device according to a comparative example conceived of by the inventor.

As illustrated in FIG. 6, the substance-in-blood concentration measurement device according to the comparative example includes a two-dimensional image-capturing means 71A that is capable of being disposed so that the relative positional relationship thereof relative to the specific region that is to become the measurement-target portion Mp is equivalent to that of the photodetector 30, and that receives reflected light reflected from the specific region and detects whether or not a focused image based on the reflected light is formed.

The two-dimensional image-capturing means 71A is a two-dimensional infrared image-capturing element array in which a plurality of light receiving elements capable of detecting mid-infrared light are arranged in a matrix on a light receiving surface 71Aa. As illustrated in FIG. 6, the two-dimensional image-capturing means 71A is integrated with the photodetector 30 to form a light detection unit 70A, and the light detection unit 70A is capable of sliding in a direction perpendicular to an optical path of the reflected light L2 from the subject portion Mp0 to the imaging lens 40. The two-dimensional image-capturing means 71A is disposed so that, when the two-dimensional image-capturing means 71A is positioned on the optical path of the reflected light L2, the relative positional relationship of the light receiving surface 71Aa of the two-dimensional image-capturing means 71A relative to the specific region is equivalent to the relative positional relationship of the screen 30a of the photodetector 30 relative to the specific region.

According to this structure, in the substance-in-blood concentration measurement device according to the comparative example, the process of adjusting the length of the optical path from the specific region to the photodetector 30 so that the reflected light from the specific region that is to become the measurement-target portion Mp forms an image on the photodetector 30 can be performed by replacing the photodetector 30 with the two-dimensional image-capturing means 71A, and thus the adjustment of optical-path length can be performed easily.

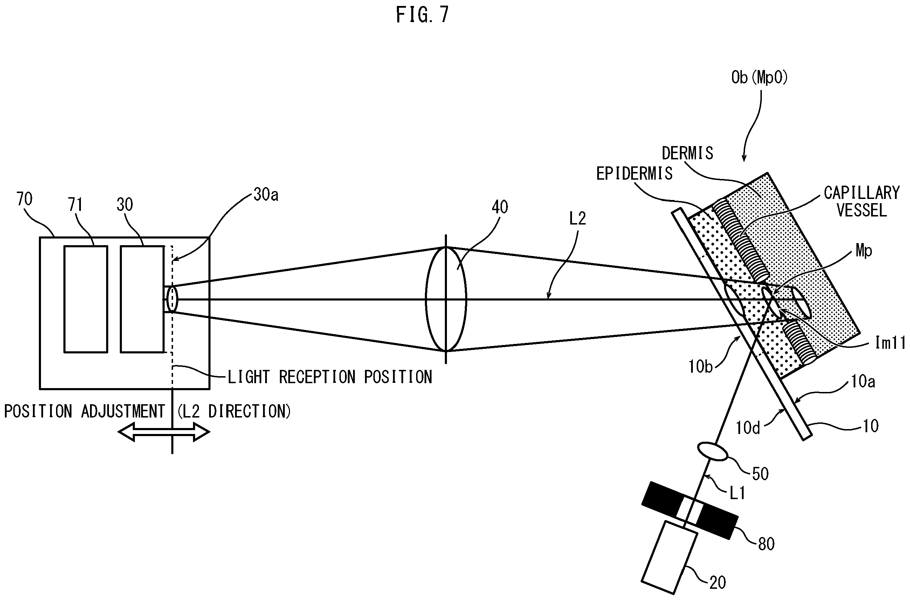

On the other hand, FIG. 7 is a schematic diagram for describing an operation, by the device 1, for adjusting the length of an optical path from the specific region (measurement-target portion Mp) to the photodetector 30.

As illustrated in FIG. 7, in the device 1, in a state in which the laser beam for reference measurement (second laser beam) L1 is emitted from the light emission unit 20, the detector 30 is moved by the movable mechanism 71 along the optical path Op2 of the reflected light L2 from the subject portion Mp0 to the photodetector 30 to change the distance of the photodetector 30 from the imaging lens 40.

According to this structure, a function is realized of changing the position of the specific region in the subject portion Mp0 from which the reflected light forming an image on the screen 30a of the detector 30 is produced. Here, the specific region is the focus region in the subject portion Mp0 where the imaging lens 40 is in focus with. This function allows the position of the photodetector 30 for measuring the measurement-target substance (first substance in blood) to be adjusted to a state in which focus is on a capillary-vessel position in the living body.

That is, the device 1 can stably measure whether or not an image Im11 based on reflected light from a blood vessel that is to become the measurement-target portion Mp is imaged as a focused image on the photodetector 30 by the reference measurement. That is, the device 1 can stably measure whether or not the image Im11 is imaged as a focused image on the photodetector by, in a state in which the laser beam for reference measurement (second laser beam) L1 is emitted, receiving the reflected light L2 reflected from the specific region and measuring the concentration of the reference substance (second substance in blood). The reference substance (second substance in blood) is characterized in that the reference substance (second substance in blood) has a higher laser-beam absorption rate than the measurement-target substance (first substance in blood) and/or the concentration in blood of the reference substance (second substance in blood) is more stable than that of the measurement-target substance (first substance in blood).

Accordingly, reflected light from a blood vessel can be detected based on reflected light produced from the laser beam for target measurement (first laser beam) while gradually moving the detector 30.

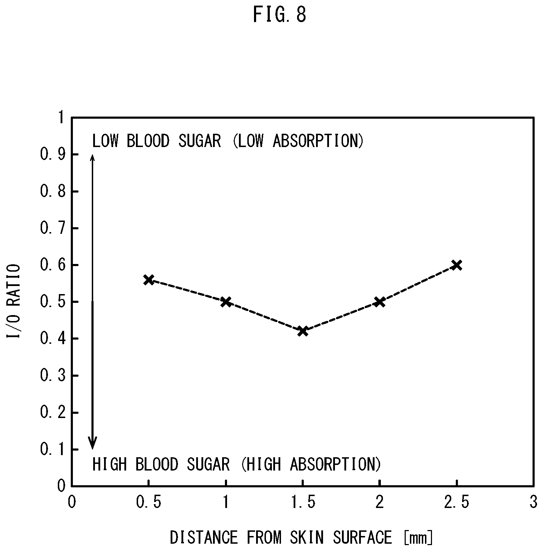

FIG. 8 is a diagram illustrating a relationship between photodetector positions and hemoglobin-concentration measurement values measured using an example of the device 1. The results are those obtained through an experiment carried out under a condition in which the laser beam for reference measurement (second laser beam) is emitted from the light emission unit 20. In FIG. 8, the horizontal axis indicates the depth of the specific region from the skin surface of the living body Ob, and the vertical axis indicates the ratio (may be referred to hereinafter as "I/O ratio") of the intensity of the reflected light L2 received by the detector 30 to the intensity of the laser beam L1 emitted from the light emission unit 20 after passing through the diaphragm 80.

As illustrated in FIG. 8, the I/O ratio exhibits the minimum value when the depth of the specific region from the skin surface of the living body Ob is 1.5 mm; thus, it can be understood that a blood-vessel region in which the hemoglobin concentration exhibits a high value is present at the depth of 1.5 mm, where the laser beam for reference measurement (second laser beam) is absorbed by hemoglobin to a great extent.

By emitting the laser beam for target measurement (first laser beam) from the light emission unit 20 and receiving the reflected light from the specific region using the detector 30 at a position of the detector 30 where reflected light from a blood vessel was detected in the above-described manner, the concentration of the measurement-target substance (first substance in blood) can be measured in a state in which the specific region is included in a blood-vessel region of the subject portion (see the annotation in FIG. 8).

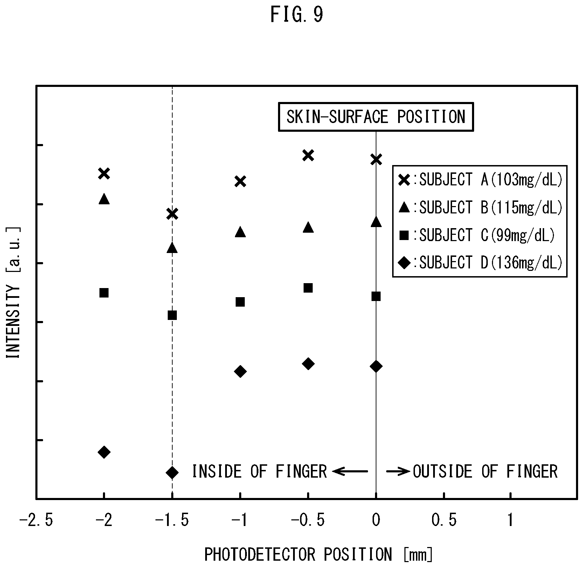

Next, as a verification experiment, the concentration in blood of glucose, which is the measurement-target substance, was measured using the example of the device 1. FIG. 9 is a diagram illustrating a relationship between photodetector positions (measurement depths) and glucose-concentration measurement values measured using the example of the device 1. Similarly to FIG. 8, in FIG. 9, the horizontal axis indicates the depth of the specific region from the skin surface of the living body Ob, and the vertical axis indicates the I/O ratio of the intensity of the reflected light L2 received by the detector 30 to the intensity of the laser beam L1 (after passing through the diaphragm 80). The parenthesized numerical value for each subject is the result of SMBG-based blood-sugar measurement carried out simultaneously with the laser measurement.

As illustrated in FIG. 9, results similar to the relationship between photodetector position and hemoglobin concentration illustrated in FIG. 8 were obtained also for glucose, which is the measurement-target substance, and, in this test, the I/O ratio exhibited the minimum value when the depth of the specific region from the skin surface of the living body Ob was 1.5 mm, also in the case of glucose. That is, according to the results of this test, it can be understood that a blood-vessel region in which the glucose concentration exhibits a high value is present at the depth of 1.5 mm, where the absorption of the laser beam for reference measurement (second laser beam) by hemoglobin exhibited a high value, and the depth where the I/O ratio exhibits the minimum value is similarly 1.5 mm regardless of the subject.

Note that, in the results illustrated in FIG. 9, the depth where the I/O ratio exhibited the minimum value was 1.5 mm in all subjects. This depth however may vary depending on lancet puncture depth and individual differences between subjects, such as the difference between an infant and an elderly person, for example. However, even in such a case, a blood-vessel region can be detected by searching for a depth where the I/O ratio exhibits a minimum value through the reference measurement.

Furthermore, according to the SMBG measurement results and laser measurement results for individual subjects, the subjects for which the amount by which the laser-intensity I/O ratio changes relative to measurement depth is greater have higher SMBG-based blood sugar measurement values, and it could thus be understood that there is a relation of dependence between the blood sugar level value and the change in laser reception intensity.

Thus, accurate measurement can be performed stably at all times regardless of the state of the skin surface, which differs between subjects and instances of measurement, and differences between individuals in the position of blood vessels in the depth direction from the skin surface. Furthermore, an effect can be obtained that measurement can be performed also on targets having thick skin by changing the light-receiving-side optical path length L by adjusting photodetector position.

### (Reduction of Measurement Fluctuation by Diaphragm 80)

An improvement, by the optical system in the device 1, of a fluctuation in substance-in-blood-concentration measurement results has been examined.

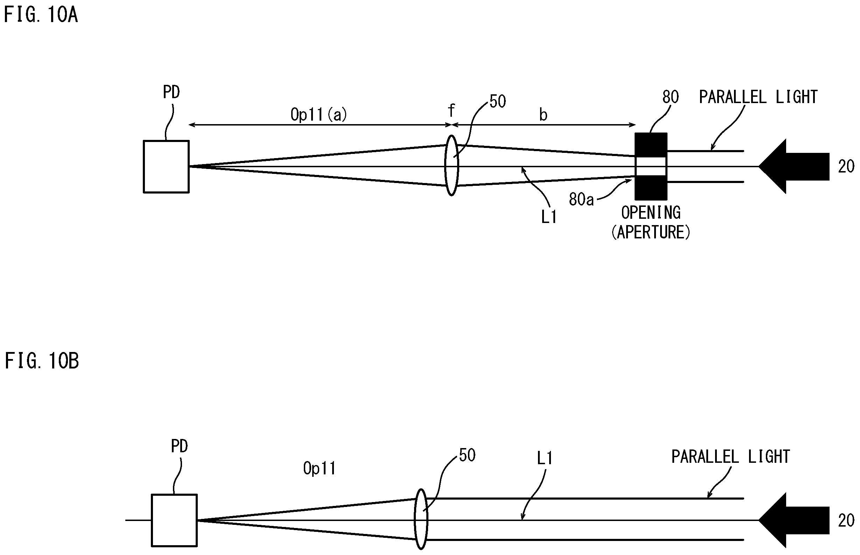

FIG. 10A is a schematic diagram for describing an overview of an optical path from the light emission unit 20 to a photodetector PD simulating the subject portion Mp0 in the substance-in-blood concentration measurement device 1, and FIG. 10B is a schematic diagram for describing the same according to a comparative example.

As illustrated in FIG. 10A, in the device 1, the diaphragm 80 is disposed in the section between the light emission unit 20 and the condenser lens 50 of an optical path of the laser beam L1 from the light emission unit 20 to the photodetector PD. The beam diameter of the laser beam L1 is restricted to one third by the opening 80a. Furthermore, the diaphragm 80 is disposed at a position where the distance between the condenser lens 50 and the light emission unit 20 is internally divided to a:b, where 1/f = 1/a + 1/b holds true, and f is the focal length of the condenser lens 50.

On the other hand, in a comparative example of the device 1, the collimated laser beam L1 is condensed to the photodetector PD by the condenser lens 50, as illustrated in FIG. 10B.

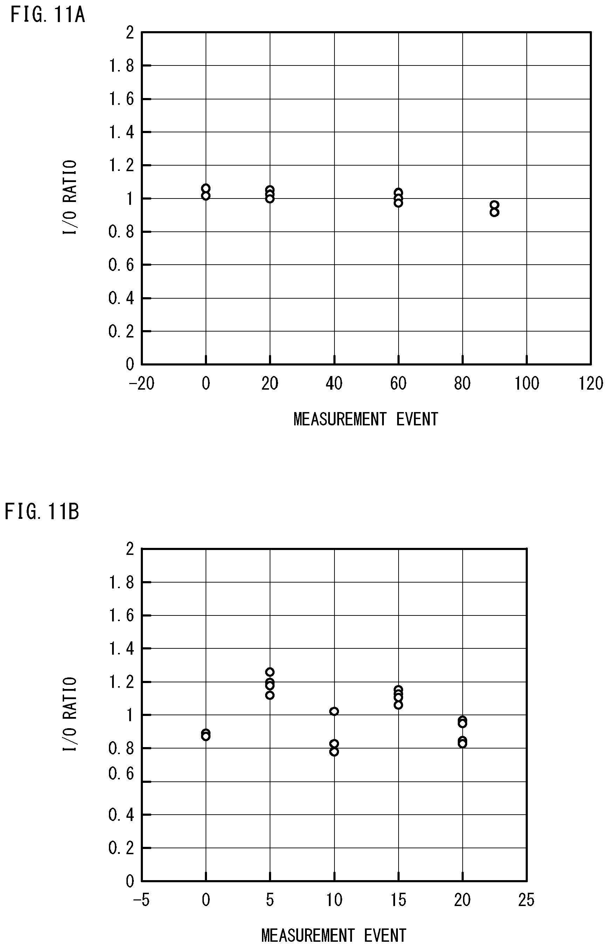

FIG. 11A is a diagram illustrating a fluctuation in substance-in-blood-concentration measurement results in the substance-in-blood concentration measurement device 1, and FIG. 11B is a diagram illustrating the same in the substance-in-blood concentration measurement device according to the comparative example. In FIGS. 11A and 11B, measurement events carried out at different times are classified from one another along the horizontal axis, and the plots in the drawings indicate measurement data items obtained by consecutively performing measurement four times in each measurement event. The vertical axis indicates numerical values obtained by normalizing the ratio of the intensity of the reflected light L2 received by the photodetector PD to the intensity of the laser beam L1 emitted from the light emission unit 20 after passing through the diaphragm 80.

As illustrated in FIGS. 11A and 11B, the fluctuations in measurement results within individual measurement events decreased from 14% to 5.3% in the device 1 compared to the comparative example, and the fluctuations in measurement results between measurement events, which was 13% at maximum in the comparative example, decreased so as to be all within the range of 3% or less in the device 1.

That is, in the device 1, in which the diaphragm 80 was provided in the optical path from the light emission unit 20 to the photodetector PD, a remarkable improvement was observed within measurement events and between events compared to the comparative example, in which the diaphragm 80 was not provided.

It can be considered that this is because, due to the provision of the diaphragm 80, beam diameter was made uniform regardless of individual differences in the size of the portion of the light emission unit 20 from which the laser beam L1 is emitted, and the center of the optical axis of the laser beam L1 was aligned with the optical axis of the condenser lens 50.

### (Reduction of Measurement Fluctuation by Measuring Inside of Skin of Living Body Ob)

A reduction of measurement fluctuation achieved by the optical system of the device 1 measuring the inside of the skin of the living body Ob has been examined.

Using the device 1, the concentration of the reference substance (second substance in blood) as measured by the photodetector 30 was measured in a comparative example in which the position of the photodetector 30 was adjusted so that the specific region is set to the skin surface of the living body Ob and an example of the device 1 in which the position of the photodetector 30 was adjusted so that the specific region is set in the skin of the living body Ob.

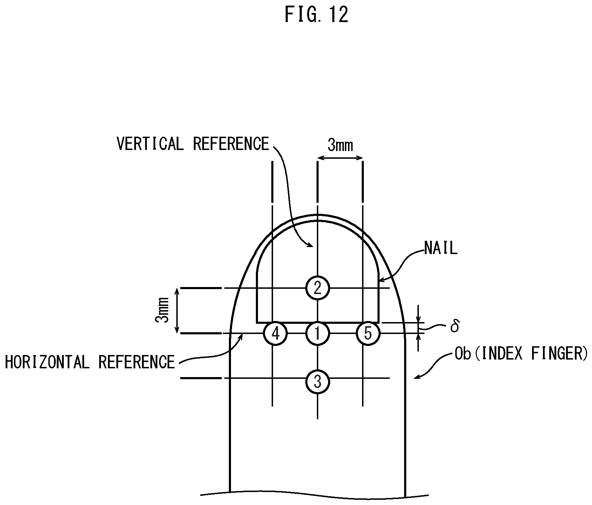

FIG. 12 is a diagram illustrating laser-beam emission positions on the surface of a living body in substance-in-blood-concentration measurement by the device 1. The index finger was used as the living body Ob, and the concentration of the reference substance was measured at a measurement position 1 on a center line toward the fingertip and a cutting line that is shifted by δ toward the finger-root direction from the root of the nail, measurement positions 2 and 3 that are on the center line and respectively offset by 3 mm upward and downward in the drawing, and measurement positions 4 and 5 that are on the cutting line and respectively offset by 3 mm toward the left and right in the drawing.

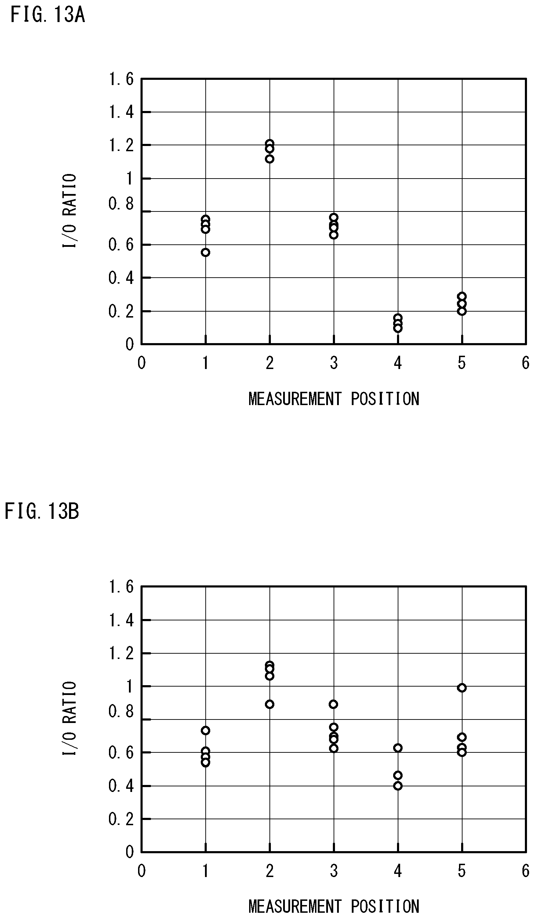

FIGS. 13A and 13B are diagrams illustrating a fluctuation in reference-substance-concentration measurement results in the example and the comparative example, respectively. In FIGS. 13A and 13B, the horizontal axis indicates the numbers of measurement positions illustrated in FIG. 12, and the plots in the drawing are measurement data items obtained by consecutively performing measurement four times at each measurement position. The vertical axis indicates numerical values obtained by normalizing the ratio of the intensity of the reflected light L2 received by the photodetector PD to the intensity of the laser beam L1 emitted from the light emission unit 20 after passing through the diaphragm 80.

As illustrated in FIGS. 13A and 13B, in the example, the fluctuation in measurement results within measurement events decreased from 40% in average in the comparative example to approximately 32% in average with the difference being 9%.

Furthermore, as illustrated in FIGS. 13A and 13B, the I/O ratio exhibited small values at measurement positions 4 and 5 in both the example and the comparative example, and it can thus be understood that a blood-vessel region exhibiting a high hemoglobin concentration value is present at measurement positions 4 and 5, where the laser beam is absorbed by hemoglobin to a great extent. In particular, regarding the relationship between I/O ratios at different measurement positions, a prominent trend in the degree of fluctuation in measurement results was observed in the example compared to the comparative example.

Furthermore, by emitting the laser beam for target measurement (first laser beam) from the light emission unit 20 at an in-plane-direction measurement position where reflected light indicating a blood-vessel region was detected, and receiving reflected light at the measurement position using the detector 30, the concentration of the measurement-target substance can be measured in a state in which the in-plane-direction measurement position is included in a blood-vessel region in the subject portion.

Thus, accurate measurement can be performed stably at all times regardless of differences between individuals in in-plane-direction positions of blood vessels, which differs between subjects and instances of measurement.

### <Operation of Substance-In-Blood Concentration Measurement Device 1>

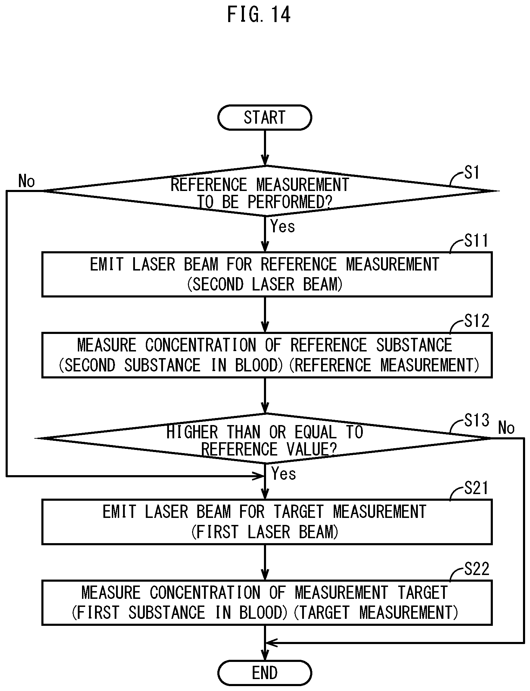

An outline of operation of the substance-in-blood concentration measurement device 1 will be described with reference to FIGS. 14, 15, and 16.

### (Operation for Determining Whether Specific Region Is Included in Blood-Vessel Region in Subject Portion)

FIG. 14 is a flowchart illustrating one structure of a substance-in-blood measurement operation by the device 1.

In FIG. 14, steps S11 and S12 are steps for performing the reference measurement, in which the concentration of the reference substance (second substance in blood) in the specific region is measured, and it is determined whether or not the specific region is included in a blood-vessel region in the subject portion.

Steps S21 and S22 are steps for the target measurement, in which the concentration of the measurement-target substance (first substance in blood) is measured in a state in which the specific region is included in a blood-vessel region in the subject portion.

In FIG. 14, first, it is determined whether or not the reference measurement is to be performed (step S1). This determination may be carried out based on input of an operation by an operator or identification information such as the ID of the subject. For example, in a case in which the same subject is measured repetitively on a daily basis, reference measurement results that have already been acquired can be used, and thus the reference measurement can be skipped. Processing proceeds to step S11 if the result of the determination in step S1 is that the reference measurement is to be performed (step S1: Yes), and processing proceeds to step S21 if the result of the determination in step S1 is that the reference measurement is not to be performed (step S1: No).

Next, the reference measurement is performed in steps S11 and S12. First, based on a control signal, the measurement controller 60 emits the laser beam for reference measurement (second laser beam) from the light emission unit 20 to the specific region (step S11), measures the concentration of the reference substance (second substance in blood) in the specific region using the photodetector 30 (step S12), and determines whether or not the measured concentration of the reference substance is higher than or equal to a reference value (step S13).

Processing is terminated if the measured concentration is not higher than or equal to the reference value in step S13 (step S13: No), whereas, the concentration of the measurement-target substance (first substance in blood) is measured in steps S21 and S22 if the measured concentration is higher than or equal to the reference value in step S12 (step S13: Yes) because it is determined that the specific region is included in a blood-vessel region in the subject portion. Specifically, the laser beam for target measurement (first laser beam) is emitted from the light emission unit 20 to the specific region (step S21), a measurement of the concentration of the measurement-target substance in the specific region (target measurement) is performed using the photodetector 30 to output a measurement result (step S22), and processing is terminated.

### (Operation for Adjusting Photodetector Position along the Optical Path)

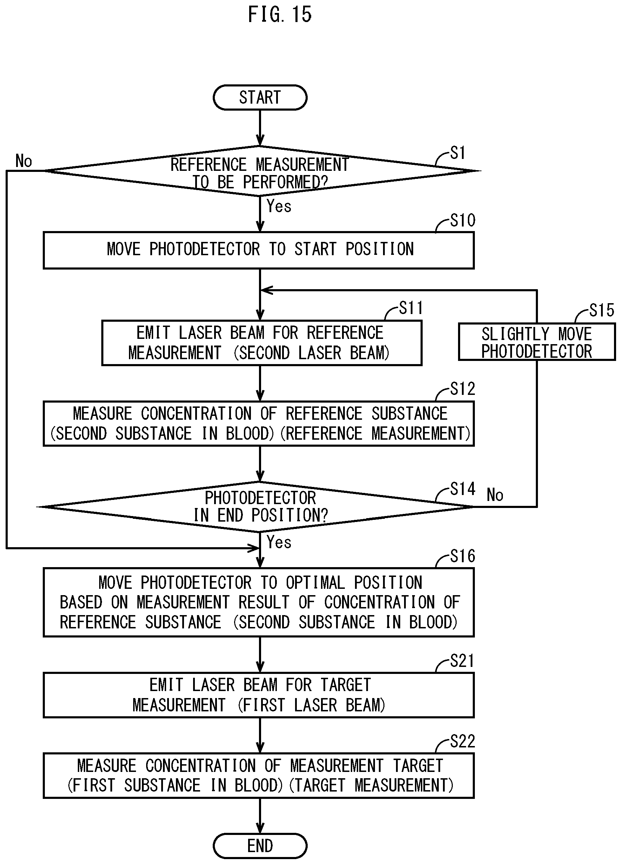

FIG. 15 is a flowchart illustrating another structure of the substance-in-blood measurement operation by the substance-in-blood concentration measurement device 1.

In FIG. 15, Steps S10 to S16 are steps for measuring the concentration of the reference substance (second substance in blood) in the specific region as the reference measurement, and adjusting the position of the photodetector along the optical path based on the concentration of the reference substance in the specific region so that the specific region is included in a blood-vessel region in the subject portion.

Steps S21 and S22 are steps for the target measurement, in which the concentration of the measurement-target substance (first substance in blood) is measured in a state in which the specific region is included in a blood-vessel region in the subject portion. Processing that is the same as processing in FIG. 14 is indicated with the same number as that in FIG. 14.

In FIG. 15, if the result of the determination in step S1 is that the reference measurement is to be performed (step S1: Yes), the reference measurement is performed in steps S10 to S16. First, based on a control signal, the measurement controller 60 drives the movable mechanism 71 to move the photodetector 30 to a start position (step S10), emits the laser beam for reference measurement (second laser beam) from the light emission unit 20 to the specific region (step S11), and measures the concentration of the reference substance (second substance in blood) in the specific region using the photodetector 30 (reference measurement; step S12).

Next, it is determined whether or not the photodetector 30 is in an end position (step S14). If the photodetector 30 is not in the end position (step S14: No), the position of the photodetector 30 is moved slightly (step S15), and processing returns to step S11, whereas, if the photodetector 30 is in the end position (step S14: Yes), processing proceeds to step S16, it being regarding that the measurement of the concentration of the reference substance in the specific region has been performed at all photodetector positions.

In step S16, based on the results of the measurement of the concentration of the reference substance in the specific region at all photodetector positions, the photodetector is moved to an optimal position where the result indicating the highest reference-substance concentration was obtained. It is inferred that the specific region is included in a blood-vessel region in the subject portion at the optimal position.

Next, the concentration of the measurement-target substance (first substance in blood) is measured in steps S21 and S22. Specifically, the laser beam for target measurement (first laser beam) is emitted from the light emission unit 20 to the specific region (step S21), a measurement (target measurement) of the concentration of the measurement target in the specific region is performed using the photodetector 30 to output a result (target measurement; step S22), and processing is terminated.

### (Operation for Adjusting In-Plane-Direction Emission Position of Laser Beam L1)

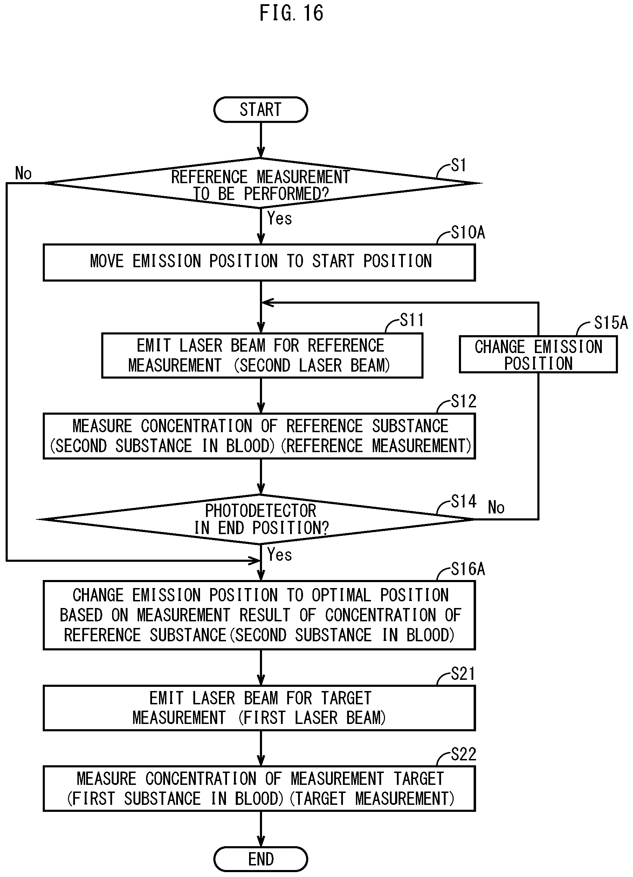

FIG. 16 is a flowchart illustrating yet another structure of the substance-in-blood measurement operation by the substance-in-blood concentration measurement device 1.

In FIG. 16, steps S10A to S16A are steps for measuring the concentration of the reference substance (second substance in blood) in the specific region while changing the in-plane direction position to which the laser beam L1 is emitted as the reference measurement, and, based on the concentration of the reference substance at different emission positions, adjusting the in-plane-direction emission position so as to be included in a blood-vessel region in the in-plane direction in the subject portion.

Steps S21 and S22 are steps for the target measurement, in which the concentration of the measurement-target substance (first substance in blood) is measured in a state in which the emission position is included in a blood-vessel region in the in-plane direction in the subject portion.

Processing that is the same as processing in FIGS. 14 and 15 is indicated with the same number.

In FIG. 16, if the result of the determination in step S1 is that the reference measurement is to be performed (step S1: Yes), the reference measurement is performed in steps S10A to S16A. First, based on a control signal, the measurement controller 60 moves the in-plane-direction position to which the laser beam L1 is emitted to a start position (step S10A), emits the laser beam for reference measurement (second laser beam) from the light emission unit 20 to the specific region (step S11), and measures the concentration of the reference substance (second substance in blood) in the specific region using the photodetector 30 (step S12).

Next, it is determined whether or not the emission position is in an end position (step S14). If the emission position is not in the end position (step S14: No), the emission position is changed (step S15A), and processing returns to step S11, whereas, if the emission position is in the end position (step S14: Yes), processing proceeds to step S16A, it being regarded that the measurement of the concentration of the reference substance in the specific region has been performed at all emission positions.

In step S16A, based on the results of the measurement of the concentration of the reference substance (second substance in blood) in the specific region at all emission positions, the emission position is changed to an optimal position where the result indicating the highest reference-substance concentration was obtained. It is inferred that the emission position is included in a blood-vessel region in the in-plane direction of the subject portion at the optimal position.

Next, the concentration of the measurement-target substance (first substance in blood) is measured in steps S21 and S22. Specifically, the laser beam for target measurement (first laser beam) is emitted from the light emission unit 20 to the specific region (step S21), a measurement of the concentration of the measurement target in the specific region is performed using the photodetector 30 to output a result (target measurement; step S22), and processing is terminated.

### <Conclusion>

As described above, a substance-in-blood concentration measurement device 1 according to the embodiment is a substance-in-blood concentration measurement device 1 that measures a concentration of a substance in blood included in the blood in a subject portion Mp0 of a living body Ob, the substance-in-blood concentration measurement device 1 including: a light emission unit 20 that emits a laser beam to a region of the subject portion that includes a measurement-target portion Mp; a photodetector 30 that receives reflected light L2 based on the emitted laser beam L1 and detects an intensity of the reflected light; a first lens 40 that is disposed between the subject portion Mp0 and the photodetector 30 in a position where the reflected light L2 from a specific region Mp in the subject portion Mp0 can form a focused image on the photodetector 30; and a measurement controller 60 that, based on the intensity of the reflected light L2, measures a concentration of the substance in blood in the specific region Mp as a concentration of the substance in blood in the measurement-target portion Mp, the substance-in-blood concentration measurement device 1 being characterized in that the light emission unit 20 is capable of selectively emitting: a laser beam for target measurement that is absorbed by a measurement-target substance (first substance in blood); and a laser beam for reference measurement (second laser beam) that is absorbed by a reference substance (second substance in blood).

Furthermore, an absorption rate at which the laser beam in the reference measurement (second laser beam) is absorbed by the second substance in blood may be higher than an absorption rate at which the laser beam for target measurement is absorbed by the first substance in blood in the target measurement. Furthermore, a concentration of the reference substance in blood may be more stable than a concentration of the measurement-target substance in blood.

Furthermore, the measurement controller 60 may be capable of measuring, based on emission of the laser beam for reference measurement (second laser beam), a concentration of the reference substance (second substance in blood) in a state in which the specific region Mp is included in a blood-vessel region in the subject portion; and may be capable of measuring, based on emission of the laser beam for target measurement, a concentration of the measurement-target substance (first substance in blood) in the specific region Mp.

Furthermore, a depth of the specific region Mp in the living body may be changed by moving the photodetector 30 along an optical path Op2 from the subject portion Mp0 to the photodetector 30, and a position of the photodetector 30 may be adjusted along the optical path Op2 based on a concentration of the reference substance (second substance in blood) so that the specific region Mp is included in a blood-vessel region in the subject portion Mp0.

According to such structures, accurate measurement can be stably performed regardless of differences between individuals in the depth of the measurement-target portion in the living body and the fluctuation in laser-beam emission conditions. Thus, a noninvasive and simple measurement method can be realized by eliminating work for adjusting the optical system appropriately for each individual living body and instance of measurement in the measurement of the blood sugar level performed by patients themselves on a daily basis.

### <<Modifications>>

Up to this point, specific structures of the present disclosure have been described taking embodiments as examples, but the present disclosure is not limited by the above embodiments in any way, except for essential characteristic constituent elements thereof. For example, an embodiment that can be obtained by applying various modifications to the embodiments, and an embodiment that can be realized by combining constituent elements and functions in the embodiments within the scope of the present invention are also included in the present disclosure.

(1) In the embodiments, an embodiment has been described taking glucose as an example of the measurement-target substance (first substance in blood) to be detected by the substance-in-blood concentration measurement device. However, components in blood that can be detected by the substance-in-blood concentration measurement device according to the present disclosure are not limited to the above, and the device can be widely used for other detection targets by changing the wavelength of the laser beam L1 emitted by the light emission unit 20 according to the type of component in blood.
   For example, the wavelength of the laser beam L1 emitted by the light emission unit 20 may be 8.23 ± 0.05 µm (8.18-8.28 µm, inclusive), and the component in blood may be lactic acid. Alternatively, the wavelength may be within the range of -0.05 µm to +0.05 µm, inclusive, from 5.77 µm, 6.87 µm, 7.27 µm, 8.87 µm, or 9.55 µm. The inventor, etc., have confirmed through experimentation that lactic-acid concentration measurement values obtained by the photodetector when the wavelength of light emitted by the light emission unit 20 is set to 8.23 µm are generally correlated with lactic-acid-value measurement results obtained by SMBG.
(2) In the embodiments, an embodiment has been described taking hemoglobin as an example of the reference substance (second substance in blood) detected in the reference measurement. However, the reference substance according to the present disclosure is not limited to the above, and application to other reference substances is possible by changing the wavelength of the laser beam L1 emitted by the light emission unit 20 according to the type of component in blood to be used as the reference substance.
(3) In the embodiments, the wavelength of the oscillated laser beam L1 can be switched and adjusted by adjusting the type and matching conditions of the nonlinear optical crystal 223 in the OPO 22.

However, a device that can measure a plurality of types of components in blood may be realized by adopting, in the light emission unit 20, a device structure such that a plurality of OPOs 22 can be selectively used, and laser beams L1 of a plurality of wavelengths can be selectively emitted. Light emitted from the light source 21 may be switchingly made to enter the plurality of OPOs emitting light of different wavelengths so that light of different wavelengths is selectively emitted from the OPOs, and the reference measurement and the main measurement of the measurement target can be selectively performed using the different wavelengths.

Alternatively, a plurality of light emission units 20 emitting light of different wavelengths may be used, and the light from two light emission units 20 may be selectively emitted as the laser beam L1 using an optical coupler, a mirror, etc. Differing from a structure in which optical systems having different optical path width, thickness, etc., are used depending on the wavelengths of light, this enables the same optical system formed from the condenser lens 50, the target placement unit 10, the imaging lens 40, and the photodetector 30 capable of detecting mid-infrared light to be used as an optical system for a plurality of components in blood, for example.

(4) In the embodiments, in regard to the light emission unit 20, the first laser beam for target measurement and the second laser beam for reference measurement have different wavelengths. However, as long as the first laser beam for target measurement and the second laser beam for reference measurement are respectively absorbed by the measurement-target substance and the reference substance, an emission condition other than wavelength may be varied between the first laser beam and the second laser beam. For example, the intensity of light emitted from the emission unit may be varied between the first laser beam and the second laser beam.

(5) In the embodiments, the measurement-target substance and the reference substance are different substances in blood. However, the reference measurement may be performed using the same substance as the measurement-target substance.

(6) In the embodiments, an embodiment of a substance-in-blood concentration measurement device has been described taking as an example an optical system including the imaging lens 40 between the measurement-target portion Mp and the photodetector 30. However, as long as the substance-in-blood concentration measurement device according to the present disclosure forms an image from the reflected light L2 reflected from the measurement-target portion Mp of the living body Ob on the photodetector 30, the structure of the light-receiving-side optical system may be changed, as appropriate. For example, a structure in which a plurality of lenses are used or a structure in which a mirror is disposed in the middle of the optical path may be adopted.

(7) The order in which steps in processing are executed in the embodiment is an example for specifically describing the present invention, and an order other than the order above may be adopted. Furthermore, some of the steps in processing may be executed at the same time as (in parallel with) other steps.

Furthermore, at least some of the functions in the embodiments and the modifications thereof may be combined with one another.

### <<Supplement>>

The embodiments described above each indicate one preferred specific example of the present invention. The numerical values, the shapes, the materials, the constituent elements, the positions of arrangement and connections of constituent elements, the processes, the order of processes, etc., indicated in the embodiments are examples, and are not intended to limit the present invention. Furthermore, among the constituent elements in the embodiments, those that are not recited in the independent claims, which represent the highest-level concepts of the present invention, are described as optional constituent elements constituting preferred embodiments.

Furthermore, the order according to which the above-described method is executed is an example for specifically describing the present invention, and an order other than the order above may be adopted. Furthermore, parts of the above-described method may be performed at the same time as (in parallel with) other methods.

Furthermore, in order to facilitate understanding of the invention, constituent elements in the drawings mentioned in the embodiments are not necessarily drawn to scale. Furthermore, the present invention is not limited by the description of the above embodiments, and can be modified, as appropriate, within the scope of the present invention.

Furthermore, all numbers used above are examples for specifically describing the present invention, and the present invention is not limited by the numbers used as examples.

### [Industrial Applicability]

The substance-in-blood concentration measurement device and the substance-in-blood concentration measurement method according to aspects of the present disclosure can be widely used in medical devices for the daily measurement of states of substances in blood, such as the blood sugar level and blood lipid level, in the prevention and treatment of lifestyle-related diseases.

### [Reference Signs List]

- 1: Substance-in-blood concentration measurement device
- 10, 10X: Target placement unit
- 20, 20X: Light emission unit
- 21: Light source
- 22: Optical parametric oscillator
- 221: Incident-side semi-transmissive mirror
- 222: Output-side semi-transmissive mirror
- 223: Nonlinear optical crystal
- 30: Photodetector
- 40: Imaging lens (first lens)
- 50: Condenser lens (second lens)
- 60: Measurement controller
- 70, 70A: Light detection unit
- 71: Movable mechanism
- 71A: Two-dimensional image-capturing means
- 80: Diaphragm
- 80a: Opening

## Claims

1. A substance-in-blood concentration measurement device that measures a concentration of a substance in blood included in the blood in a subject portion of a living body, the substance-in-blood concentration measurement device comprising:
a light emission unit that emits a laser beam to a region of the subject portion that includes a measurement-target portion;
a photodetector that receives reflected light based on the emitted laser beam and detects an intensity of the reflected light;
a first lens that is disposed between the subject portion and the photodetector in a position where the reflected light from a specific region in the subject portion can form a focused image on the photodetector; and
a measurement controller that, based on the intensity of the reflected light, measures a concentration of the substance in blood in the specific region as a concentration of the substance in blood in the measurement-target portion, wherein
the light emission unit is capable of selectively emitting: a first laser beam for a target measurement that is absorbed by a first substance in blood that is a measurement-target substance; and a second laser beam for a reference measurement that is absorbed by a second substance in blood that is a reference substance.

2. The substance-in-blood concentration measurement device according to claim 1, wherein
an absorption rate at which the second laser beam is absorbed by the second substance in blood in the reference measurement is higher than an absorption rate at which the first laser beam is absorbed by the first substance in blood in the target measurement.

3. The substance-in-blood concentration measurement device according to claim 1 or 2, wherein
a concentration of the reference substance in blood is more stable than a concentration of the measurement-target substance in blood.

4. The substance-in-blood concentration measurement device according to any one of claims 1 to 3, wherein
the measurement controller is capable of measuring, based on emission of the second laser beam, a concentration of the second substance in blood in a state in which the specific region is included in a blood-vessel region in the subject portion, and
is capable of measuring, based on emission of the first laser beam, a concentration of the first substance in blood in the specific region as a concentration of the first substance in blood in the measurement-target portion.

5. The substance-in-blood concentration measurement device according to any one of claims 1 to 4, wherein
a depth of the specific region in the living body can be changed by moving the photodetector along an optical path from the subject portion to the photodetector, and
a position of the photodetector can be adjusted along the optical path based on a concentration of the second substance in blood so that the specific region is included in a blood-vessel region in the subject portion.

6. The substance-in-blood concentration measurement device according to any one of claims 1 to 5, wherein
the light emission unit modulates a wavelength of the laser beam to change a type of substance in blood that can be detected.

7. The substance-in-blood concentration measurement device according to any one of claims 1 to 6, wherein
the light emission unit includes an optical transmitter that emits the first laser beam and an optical transmitter that emits the second laser beam.

8. The substance-in-blood concentration measurement device according to any one of claims 1 to 7, wherein
the substance in blood is glucose, and a wavelength of the laser beam is a predetermined wavelength selected from a range of 2.5-12 µm, inclusive.

9. The substance-in-blood concentration measurement device according to any one of claims 1 to 8, wherein
the substance in blood is hemoglobin, and a wavelength of the laser beam is a predetermined wavelength selected from a range of 5.0-12 µm, inclusive.

10. The substance-in-blood concentration measurement device according to any one of claims 1 to 9 further comprising:
a second lens that is positioned between the light emission unit and the subject portion in an optical path of the laser beam, and that condenses the laser beam to the region of the subject portion; and
a diaphragm that is positioned between the light emission unit and the second lens.

11. The substance-in-blood concentration measurement device according to any one of claims 1 to 10 further comprising
a target placement unit with which a surface of the living body is placed into contact, wherein
a through-hole is opened in the target placement unit within a region of the target placement unit with which the surface of the living body is placed into contact,
the laser beam is emitted to the surface of the living body through the through-hole, and
the reflected light is received by the photodetector through the through-hole.

12. The substance-in-blood concentration measurement device according to any one of claims 1 to 10 further comprising
a target placement unit with which a surface of the living body is placed into contact, wherein
a recess portion is formed in the target placement unit within a region of the target placement unit with which the surface of the living body is placed into contact,
the laser beam passes through the target placement unit and is emitted to the surface of the living body, and
the reflected light passes through the target placement unit and is received by the photodetector.

13. The substance-in-blood concentration measurement device according to any one of claims 1 to 12, wherein
the measurement-target portion is a blood-vessel region in the subject portion that is located inward of the epidermis, and
the first lens transfers a region of the measurement-target portion that is irradiated with the laser beam onto a light receiving surface of the photodetector.

14. The substance-in-blood concentration measurement device according to claim 8, wherein
the wavelength of the laser beam is a predetermined wavelength selected from a range of 6.0-12 µm, inclusive.

15. The substance-in-blood concentration measurement device according to any one of claims 1 to 7, wherein
the substance in blood is lactic acid, and a wavelength of the laser beam is a predetermined wavelength selected from a range of 5.0-12 µm, inclusive.

16. The substance-in-blood concentration measurement device according to claim 11 or 12, wherein
within a section from the target placement unit to the photodetector in an optical path from the subject portion to the photodetector, the reflected light propagates through a space except within a section in which the reflected light passes through the first lens, and
within a section from the light emission unit to the target placement unit in an optical path from the light emission unit to the subject portion, the laser beam propagates through a space except within a section in which the laser beam passes through the second lens.

17. A substance-in-blood concentration measurement method for measuring a concentration of a substance in blood included in the blood in a subject portion of a living body, the substance-in-blood concentration measurement method comprising:
emitting, from a light emission unit to an irradiation region in the subject portion that includes a measurement-target portion, a first laser beam for a target measurement that is absorbed by a first substance in blood that is a measurement-target substance;
forming a focused image on a photodetector from reflected light of the first laser beam reflected from a specific region in the subject portion using a first lens positioned between the subject portion and the photodetector; and
performing the target measurement by receiving the reflected light of the first laser beam using the photodetector, and measuring a concentration of the first substance in blood based on the reflected light as a concentration of the first substance in blood in the measurement-target portion, wherein
prior to the target measurement, a reference measurement is performed by:
emitting, from the light emission unit to the irradiation region, a second laser beam for the reference measurement that is absorbed by a second substance in blood that is a reference substance;
forming a focused image on the photodetector from reflected light of the second laser beam reflected from the specific region using the first lens; and
receiving the reflected light of the second laser beam using the photodetector, and measuring a concentration of the second substance in blood based on the reflected light as a concentration of the second substance in blood in the measurement-target portion.

18. The substance-in-blood concentration measurement method according to claim 17, wherein
an absorption rate at which the second laser beam is absorbed by the second substance in blood in the reference measurement is higher than an absorption rate at which the first laser beam is absorbed by the first substance in blood in the target measurement.

19. The substance-in-blood concentration measurement method according to claim 17 or 18, wherein
a concentration of the reference substance in blood is more stable than a concentration of the measurement-target substance in blood.

20. The substance-in-blood concentration measurement method according to any one of claims 17 to 19, wherein
in the reference measurement,
by measuring the concentration of the second substance in blood while changing a position of the photodetector along an optical path from the subject portion to the photodetector, the position of the photodetector is adjusted along the optical path so that the specific region is included in a blood-vessel region in the subject portion.

21. The substance-in-blood concentration measurement method according to any one of claims 17 to 20, wherein
the first laser beam and the second laser beam are emitted to the irradiation region using: a second lens that is positioned between the light emission unit and the subject portion in an optical path from the light emission unit to the subject portion, and that condenses the first laser beam and the second laser beam to the irradiation region; and a diaphragm that is positioned between the light emission unit and the second lens and that restricts light emitted from the light emission unit.

22. The substance-in-blood concentration measurement method according to any one of claims 17 to 21, wherein
a surface of the living body is placed into contact with a target placement unit,
the first laser beam and the second laser beam are emitted to the surface of the living body through a through-hole opened in the target placement unit, and
the reflected light is received by the photodetector through the through-hole.

23. A program that causes a computer to execute substance-in-blood concentration measurement processing of measuring a concentration of a substance in blood included in the blood in a subject portion of a living body, wherein
the substance-in-blood concentration measurement processing includes:
emitting, from a light emission unit to an irradiation region in the subject portion that includes a measurement-target portion, a first laser beam for a target measurement that is absorbed by a first substance in blood that is a measurement-target substance;
forming a focused image on a photodetector from reflected light of the first laser beam reflected from a specific region in the subject portion using a first lens positioned between the subject portion and the photodetector; and
performing the target measurement by receiving the reflected light of the first laser beam using the photodetector, and measuring a concentration of the first substance in blood based on the reflected light as a concentration of the first substance in blood in the measurement-target portion, wherein
prior to the target measurement, a reference measurement is performed by:
emitting, from the light emission unit to the irradiation region, a second laser beam for the reference measurement that is absorbed by a second substance in blood that is a reference substance;
forming a focused image on the photodetector from reflected light of the second laser beam reflected from the specific region using the first lens; and
receiving the reflected light of the second laser beam using the photodetector, and measuring a concentration of the second substance in blood based on the reflected light as a concentration of the second substance in blood in the measurement-target portion.

24. The program according to claim 23, wherein
in the reference measurement,
by further measuring the concentration of the second substance in blood while changing a position of the photodetector along an optical path from the subject portion to the photodetector, the position of the photodetector is adjusted along the optical path so that the specific region is included in a blood-vessel region in the subject portion.
